(19) **Europäisches Patentamt**

**European Patent Office**

**Office européen des brevets**

(11) Publication number: **0 419 081 A2**

## EUROPEAN PATENT APPLICATION

(21) Application number: **90309624.6**

(22) Date of filing: **03.09.90**

(51) Int. Cl.5: **G01N 33/58**, G01N 33/535, G01N 33/542, C12N 9/38, C12N 15/56, C12N 1/20, //(C12N1/20,C12R1:19)

(30) Priority: **22.09.89 US 410996**

(43) Date of publication of application:
**27.03.91 Bulletin 91/13**

(84) Designated Contracting States:
**AT BE CH DE DK ES FR GB GR IT LI LU NL SE**

(71) Applicant: **MICROGENICS CORPORATION**
**2380A Bisso Lane**
**Concord California 94520(US)**

(72) Inventor: **Henderson, Daniel R.**
**1196 Grove Circle**
**Benicia, California 94510(US)**

(74) Representative: **Harrison, David Christopher**
**et al**
**MEWBURN ELLIS 2 Cursitor Street**
**London EC4A 1BQ(GB)**

(54) Method for protein binding enzyme complementation assays.

(57) This invention relates to improved methods and novel compositions for enzyme complementation assays for qualitative and quantitative determination of a suspected analytein a sample. The use of enzyme-acceptor and enzyme-donor polypeptides prepared by recombinant DNA techniques or chemical polypeptide synthesis techniques which are capable of interacting to form an active enzyme complex having catalytic activity characteristic of β-galactosidase is described. Both homogeneous and heterogeneous assays utilizing these polypeptides are describe.

EP 0 419 081 A2

## METHOD FOR PROTEIN BINDING ENZYME COMPLEMENTATION ASSAYS

The technical field of this invention is enzyme immunoassays.

The prior art teaches many immunoassays based on the pioneering development of radioimmunoassay (RIA) by Yalow and Berson, 1960, J. Clin. Invest., 39:1157. RIAs are characterized by competing fixed amounts of radio-labeled analytes with unknown quantities of unlabeled .analytes for fixed amounts of specific antibody. The amount of radioactive analyte either bound to antibody or free in solution is quantitated in an appropriate counter and the concentration of non-radioactive analyte determined. Improvements on this general scheme have included: (1) substitution of the radioactive tracer with enzyme or fluorescent tracers, (2) substitution of polyclonal animal antibodies with monoclonal antibodies, (3) improved methods of signal detection including spectrophotometers, fluorometers, fluorescence polarizers and particle counters, and (4) the introduction of homogeneous assays not requiring physical separation of bound tracer from free tracer. Separation of bound tracer from free tracer frequently requires solid supports such as plastic, paper, glass or acrylamide. Customarily, antibody is bound to the solid phase whereas tracers and unknowns are free in solution. The bound/free separation is accomplished by one or more washes of the solid phase. The residual bound activity is then measured. These assays are known collectively as heterogeneous immunoassays. In comparison, homogeneous assays obviate the need for the imprecise and time-consuming separation steps.

Commercialization of immunoassays has seen a shift in usage from radioimmunoassays, to enzyme-linked immunosorbent assays (ELISA), to homogeneous assays. This shift is due to the commercial demands of speed, simplicity, automation and absence of radioactivity. Homogeneous assays consist of several types: (1) nephelometry, (2) particle counting, (3) fluorescent quenching, (4) fluorescence polarization, and (5) enzyme assays.

The first nephelometer to measure light dispersion to quantitate immune reactions was devised in the late 1960s. These early nephelometers were improved ten years later with new chemistries, lower angles for measuring dispersion angles and the ability to measure the rate of the antigen-antibody reaction during the first seconds after mixing the reactants (Ritchie, Alper and Graves 1969, Arthritis Rheum. 12:693; Deaton et al., 1976, Clin. Chem. 22:1465). These assays are of extremely poor sensitivity and are applicable to determinations of analytes at concentrations greater than $10^{-8}M$, e.g., serum IgE, IgA and IgM levels. In homogeneous particle counting assays, polystyrene particles 0.8 $\mu m$ in diameter (latex particles) are coated by antibodies. Antigen concentrations can be determined by the concentration of latex particles agglutinated as determined by an instrument capable of distinguishing agglutinated versus nonagglutinated particles (Cambiaso et al., 1977, J. Immunol. Meth. 18:33). Homogeneous fluorescent quenching assays label either antigens or antibodies with a fluorophor. Analyte-antibody-fluorophor complexes yield significantly less fluorescence compared to the antigen-fluorophor or antibody-fluorophor alone (Ullman et al., 1979, J. Biol. Chem. 251:4172; U.S. Pat. Nos. 3,998,943; 3,996,345; 4,174,384; 4,161,515; 4,208,479 and 4,160,016). All these assays involve various methods of quenching fluorescence such that the amount of quenching is related to the amount of the unknown analyte or antibody in the sample. These assays are of low sensitivity (analytes at fluid concentrations greater than $10^{-10}M$). The low sensitivity is due to endogenous serum fluorescence and the use of fluorescence in a static non-enzymatically amplified manner. Fluorescence polarization assays are based on the free rotation of antigen-fluorophor in solution which is significantly reduced by antibody binding to the antigen-fluorophor and have found considerable commercial success with low molecular weight (under 1000 daltons molecular weight) analytes (Dandliker et al., 1973, Immunochemistry 10:219).

The various immunoassay methods each possess commercial advantages and disadvantages. RIAs are sensitive and easy to set-up but require radioactivity, separation steps and expensive instrumentation. Heterogeneous assays with enzymes or fluorophores eliminate radioactivity and some instrumentation but require separation steps. From a commercial viewpoint it is desirable to eliminate separation steps for several reasons. Separations (1) are labor intensive, (2) are time consuming, (3) require additional equipment, (4) increase variability in results, and (5) preclude high levels of automation. Despite the many commercial advantages of homogeneous immunoassays only three systems, the enzyme-labeled system of Rubenstein et al, U.S. Pat. No. 3,817,837, the substrate-labeled system of Burd et al., 1977, Clin. Chem. 23:1402, and fluorescence polarization (Dandliker et al., 1973, Immunochemistry) have found commercial success. Yet these three assay systems are limited to small (less than 1000) molecular weight analytes and analytes found in concentrations greater than $10^{-10}M$.

Relevant Literature

Enzyme immunoassays have been a very successful type of homogeneous immunoassay. Several variants of homogeneous enzyme immunoassays have found commercial success (1) the enzyme labeled analyte system; and (2) the substrate labeled analyte system. In the enzyme labeled system the enzymatic activity of the label is decreased when specific antibody binds the analyte-enzyme complex. Analyte to be measured competes with a fixed amount of specific antibody for a fixed amount of the analyte. Enzyme activity is directly proportional to the unknown analyte concentration. The following patents have been issued based on this immunoassay system: U.S. Pat. Nos. 3,817,837; 3,852,157; 3,875,011; 3,966,556; 3,905,871; 4,065,354; 4,043,872; 4,040,907; 4,039,385; 4,046,636; 4,067,774; 4,191,613 and 4,171,244. Commercialization of this technology has been limited to low molecular weight analytes and low sensitivity (analytes smaller than 1000 daltons MW at concentrations greater than analytes $10^{-10}$ M).

The substrate-labeled fluorescent immunoassay involves covalent coupling of the analyte to a fluorogenic substrate for an enzyme. This analyte-substrate conjugate is not fluorescent. In the absence of antibody the analyte-fluorogenic substrate is hydrolyzed by an enzyme yielding a fluorescent molecular species. In the presence of specific antibody, access to the substrate by the enzyme is curtailed yielding decreased fluorescence (Burd et al., 1977, Clin. Chem. 23:1402; Burd et al., Anal. Biochem. 77:56; and Kohen, Hollander and Boguslaski, 1979, J. Steriod Biochem. 11:161). Commercialization of this assay system has been limited to low molecular weight analytes due to steric considerations, and to analytes at concentrations in fluids greater than $10^{-10}$ M due to considerations analogous to those for the fluorescence quenching assays described above.

Numerous homogeneous enzyme immunoassays have been described which have encountered limited commercialization.

U.S. Pat. No. 4,134,792 describes an immunoassay technique utilizing an enzyme modulator such as an enzyme inhibitor or an allosteric effector as a label. When specific antibody binds to an enzyme modulator-labeled analyte, the enzyme modulator can no longer inhibit the activity of the enzyme. Thus, competition of the enzyme modulator-labeled analyte by free analyte restores inhibition of the enzyme modulator. Other patents in this field include: U.S. Pat. Nos. 3,935,074; 4,130,462; 4,160,645 and 4,913,983.

U.S. Pat. Nos. 4,213,893 and 4,318,983 describe enzyme-immunoassays employing cofactor-apoenzyme systems. In particular, U.S. Pat. No. 4,318,983 issued to Hornby et al. (Mar. 9, 1982) describes a method employing flavin adenine dinucleotide (FAD)-labeled conjugates and apoenzymes with which FAD acts as a prosthetic group. U.S. Pat. No. 4,213,893 issued to Carrico et al. (July 22, 1980) describes specific FAD-labeled conjugates, e.g., FAD-labeled thyroxine, which are suitable for use in the Hornby et al. method. FAD-labeled conjugates are monitored by measuring holoenzyme activity generated by incubation of such conjugate with an apoenzyme that requires FAD for catalytic activity. An analyte is covalently coupled to FAD such that the labeled cofactor retains its reactivity with dehydrogenase enzymes. The amount of reduced FAD formed by the dehydrogenase activity is decreased in the presence of antibody specific for the analyte. The fluorometrically monitored appearance of reduced FAD is directly proportional to the amount of analyte (Cohen et al., 1978, in Enzyme-labeled Immunoassay for Hormones and Drugs, S.N. Pal, ed., Walter deGuiter, Berlin and New York, pg. 67-79). A similar system for biotin and 2,4-dinitrofluorobenzene analytes using lactic dehydrogenase and diaphorase has been described (Carrico et al., 1976, Anal. Biochem. 73:271). Both systems suffer from interference from endogenous cofactors and enzymes that are common in serum samples to be analyzed.

Several enzymes have been observed to reform from peptide fragments but only a few regain enzymatic activity including, e.g., ribonuclease A (Richards and Vithayathil, 1959, J. Biol. Chem. 234:1459), staphlococcal nuclease (Light et al., 1974, J. Biol. Chem. 249:2285), and β-galactosidase (Langley and Zabin, 1976, Biochemistry 15:4866). Proteolysis of bovine pancreatic ribonuclease by subtilisin yields two components, a peptide (S-peptide) and a protein (S-protein). Neither S-peptide nor S-protein alone shows appreciable ribonuclease activity. When these components are mixed in molar equivalents, almost the full enzymatic activity is recovered. S-peptide and S- protein re-associate very rapidly and strongly with a $K_{eq} = 5 \times 10^{-9}$ M (Richards and Vithayathil, 1959, supra). Staphlococcal nuclease shows reconstruction of biologically active enzyme from inactive peptide fragments. Nuclease-T(6-48), including amino acids 6-48 of the full 149 amino acid staphlococcal nuclease structure, re-associates with Nuclease-T(50-149) to form active Nuclease-T1 with a first order rate constant of 0.03-0.05/sec with little temperature variability (Light, supra). As discussed in greater detail, polypeptide fragments (e.g., M15) from deletion mutants of E . coli are known which regain enzymatic activity when combined with small peptide fragments derived from thermally or cyanogen bromide treated β-galactozidase enzyme. One cyanogen bromide-generated fragment is called CNBr2; another is called CNBr24.

More recently, an immunoassay based on the re-association of such polypeptide fragments was described by Farina and Golke (U.S. Pat. No. 4,378,428 issued March 29, 1983) and by Gonelli et al., (1981, Biochem. and Biophys. Res. Commun. 102:917-923). All experimental examples disclosed therein were based on re-association of S-peptide/S-protein to generate ribonuclease catalytic activity. An analyte was covalently attached to a small subtilisin cleavage peptide of ribonuclease, i.e., the S-peptide (amino acids 1-20). This was coupled to an analyte and combined with S-protein (amino acids 21-124) to reform active ribonuclease. Antibody specific for the analyte inhibits the reformation of ribonuclease activity. This assay is limited due to the presence of endogenous ribonuclease activity in all non-autoclaved biological solutions.

Other equally serious faults never addressed by this system include the inability to adjust the equilibrium constant of the associating polypeptides, and an inability to create immunoreactive polypeptides which could couple to large molecular weight proteins while still capable of reforming active enzyme. All polypeptides utilized were non-novel catalytically inactive peptides capable of re-association to form active ribonuclease.

More significant disadvantages with the chemistries proposed by Farina and Golke (U.S. Pat. No. 4,378;428) to attach an analyte to CNBr2 or M15 have been discovered. Attaching an analyte through the available $NH_2$, COOH, and SH groups on either of the polypeptides have, in all cases tested, yielded polypeptides incapable of complementation. Coupling M15 which has many amino, carboxylic acid and sulfhydryl functionalities, inactivated M15 in all cases, even with carefully controlled conditions. Kinetics indicate a single hit to be sufficient to inactivate activity. CNBr2 contains no internal lysines, a single sulfhydryl group and several carboxylic acid groups. In agreement with Langley (Ph.D. thesis entitled "The Molecular Nature of $\beta$-galactosidase $\alpha$-complementation", UCLA, 1975) coupling to the N-terminal $\alpha$-amino group inactivates complementation activity of $CNBr_2$. In the preparation of CNBr2 (Langley, Fowler and Zabin, 1975, J. Biol. Chem. 250:2587), the sulfhydryl at position 76 is reduced and alkylated with iodoacetic acid prior to the cyanogen bromide cleavage. If the sulfhydryl is not alkylated CNBr2 activity can be retained early in the steps of purification but is lost prior to purification to homogeneity. Also, if the sulfhydryl is alkylated with a maleimide derivative of an analyte instead of iodoacetic acid, insolubility of the conjugate prevents purification. Finally, in all cases tested, coupling to a COOH moiety of CNBr2 inactivated complementation activity. Therefore, it appears to be difficult to use CNBr2 and M15 to prepare appropriate immunoreactive and complementing reagents.

Mutant polypeptides derived from $\beta$-galactosidase are known which can complement or spontaneously restore enzyme activity when added to extracts of appropriate $\beta$-galactosidase negative mutants. This phenomenon is known as intracistronic complementation. An example of $\alpha$-complementation is provided by the M15/CNBr2 complementation system. The M15 mutant polypeptide lacks amino acids 11-41 of $\beta$-galactosidase and exists in solution as an enzymatically inactive dimer. A polypeptide derived from $\beta$-galactosidase by cyanogen bromide (CNBr) cleavage, the $CNBr_2$ peptide ($CNBr_2$) , consists of amino acids 3-92. $CNBr_2$, when mixed with the dimer M15, promotes spontaneous reconstruction of the $\beta$-galactosidase tetramer with full enzymatic activity (Langley and Zabin, 1976, Biochemistry 15:4866). The M15 peptide is known as an $\alpha$-acceptor and CNBr2 as an $\alpha$-donor. While this represents a well-studied complementing system, CNBr2 can serve as a-donor for the M112 dimer, a deletion of amino acids 23-31 within $\beta$-galactosidase (Lin, Villarejo and Zabin, 1970, Biochem. Biophys. Res. Common. 40:249; Celeda and Zabin, 1979, Biochem. 18:404; Welphy, Powler and Zabin, 1981, J. Biol. Chem. 256:6804; Langley et al., 1975, Proc. Natl. Acad. Sci. USA 72:1254). Other $\alpha$-donors include a polypeptide derived by autoclaving $\beta$-galactosidase. This peptide, however, has not been purified and its sequence is unknown. $\alpha$-acceptors other than M15 and M112 have not been described. In the example of complementation of M15 by CNBr2, amino acid sequences 3-10 and 42-96 are both present in duplicate in the enzymatically active complex.

Intracistronic complementation also occurs at the C-terminus of $\beta$-galactosidase (the $\omega$-region). The best known sequence data available is for the X90 $\omega$-acceptor peptide that deletes the last 10 amino acids, 1011-1021. The X90 peptide exists as a monomer and can be complemented by CNBr24, a cyanogen bromide digestion product of $\beta$-amino acids 990-1023 to reform enzymatically active tetramer (Welphy et al., 1980, Biochem. Biophys. Res. Common. 93:223).

In US-A-4708929 there are disclosed enzyme-acceptor and enzyme-donor polypeptides prepared by recombinant DNA techniques or chemical polypeptide synthesis techniques which are capable of interacting to form an active enzyme complex having catalytic activity characteristic of $\beta$-galactosidase, and their application to homogeneous and heterogeneous assays.

The subject invention provides novel assays for ligands and receptors and compositions for use in the assays comprising enzyme complementation fragments, where one of the fragments is conjugated to a member of a specific binding pair, where the analyte is cross reactive with the conjugated member of the specific binding pair or is complementary to such conjugated member. The reagents are combined with the sample in an appropriate assay medium and the rate of formation of enzymatic product determined as an indication of the presence of analyte in the medium. Of particular interest are synthetic sequences which provide for the presence of an amino acid having a functionality for linking to the specific binding pair member.

In accordance with the subject invention, diagnostic assays are provided, as well as reagents for use in the diagnostic as says. The reagents comprise two complementary fragments, which when complexed provide for an active β-galactosidase enzyme. Also provided are nucleic acid sequences encoding the mutated fragments, methods for preparing the mutated fragments, including fusion proteins involving amino acid sequences defining epitopes of interest, and methods for expressing such products.

β-Galactosidase is a tetrameric protein having a molecular weight of about 540 kD. The four identical subunits or monomers consist of 1023 amino acids, each with a molecular weight of 116 kD. The monomer may be divided up into two portions, an α-portion of from about 70-100 amino acids of the N-terminus and a remaining portion of the molecule which may partially overlap the α-portion. These two molecules can be used to complement to form a complex which is an active enzyme. For the purposes of the subject invention, the α-region or N-terminal portion will be referred to as the enzyme donor (ED) and the remaining portion as the enzyme acceptor (EA). The enzyme donor will normally be the smaller fragment, except when it serves as a fusion protein. The enzyme acceptor will normally be the larger protein. For the most part, the ED will be the site of conjugation, although either fragment may be the site of conjugation.

Enzyme Donors

The natural sequence of β-galactosidase may be changed by insertions, deletions, substitutions, and combinations thereof. For the most part, a single substitution will be employed, and not more than about three substitutions, usually not more than about too substitutions. For the most part, a substitution will involve exchanging one amino acid for a different amino acid which has a functionality, such as sulfhydryl, amino, hydroxyl, or carboxyl, which allows for conjugation at that site. Preferably, the substitution will be to a cysteine or lysine.

The basic sequence which will be referred to is as follows:

```
1*      *   *   5                   10                  15
M   D   P   S   G   N   P   Y   G   I   D   P   T   Q   S

                20          *   25*                 30
    S   P   G   N   I   D   P   R   A   S   S   N   S   L   A
                                                6

            *  35                   *  40*  *   *   *   *  45*
    V   V   L   Q   R   R   D   W   E   N   P   G   V   T   Q
                                                20

    *       *   50          *       55*                 60
    L   N   R   L   A   H   P   P   F   A   S   W   R   N
                        30

    *               65          *   70                  75
    S   E   E   A   R   T   D   R   P   S   Q   Q   L   R   S
        40              .                           50

                    80                  85  *       89
    L   N   G   L   E   S   R   S   A   G   M   P   L   G
            56
```

Numbers underneath letters indicate the wild-type β-galactosidase numbering.

\* Indicates amino acid substitutions to C (Cysteine) or K (Lysine).

Preferred regions for substitutions include the region from about amino acid 1 to amino acid 30; from amino acid 35 to amino acid 45; from amino acid 60 to amino acid 89. Where more than one substitution is employed, it is preferred the substitutions be separated by at least about 5 amino acids, preferably at least about 10 amino acids, and more preferably from about 20 to 60 amino acids. Preferably, the region from about amino acids 48 to 61 are not used for substitution, although the particular site for substitution will to a significant degree depend upon the nature of the conjugate. Thus, one site may be favored over another site when preparing one conjugate as compared to another conjugate.

Sites of particular interest include amino acids 1, 3, 23, 25, 39, 42, 45, 46, 61 and 68, where the region 1 to 5 and 40 to 47, particularly 46 are sites for two or more substitions. Regions for deletion include the region from amino acids 1 to 20, particularly 5 to 20, or any sequence therein. Regions of interest for substitution of other than a conjugation site include the region from about 70 to 85, particularly from about 72 to 80, more particularly 74 to 77, where a greater or lesser number of amino acids may be introduced, where the substitutions may be conservative or non-conservative. By conservative is intended having the same or substantially the same charge type and general conformation, for example, neutral amino acids may be substituted for other neutral amino acids, aromatic amino acids for other aromatic amino acids, charged amino acids for other charged amino acids of the same charge type, and the like. Furthermore, one could consider for conservative changes, retaining a hydrophobic region as compared to a hydrophilic region, where non-conservative would be to change the nature of the region from hydrophilic to hydrophobic or vice versa.

A large number of linking groups may be employed for joining a wide variety of specific binding pair members to a functionality present in the ED. As already indicated, for the most part, the functionality present on the ED for linking will be a mercaptan or amino group. For mercaptans, of particular interest are a wide variety of readily available reagents, involving activated halogen, activated olefin, or mercapto, where the first two form thioethers and the second a disulfide. Specific compounds include N-maleimidobenzoic acid, α-bromoacetamidocyclohexanecarboxylic acid, N-maleimidosuccinic acid, methyldithioacetic acid, etc. For amino groups, a wide variety of active halogens or carboxylic acid groups may be employed, particularly activated carboxylic acid groups, where the carboxylic acid groups may be activated with carbodiimide, active esters, such as N-hydroxy succinimide, o -nitrophenol, p -nitrophenol, etc. The procedures for conjugation are well known in the literature and are amply illustrated by U.S. Patent Nos. 3,817,837; 4,262,089; 4,233,401; 4,220,722 and 4,374,925.

The linking group may merely be a bond, for example where the ligand has a carboxylic acid group which can be activated to react with the amino group of ED or may be of one or more atoms other than hydrogen, usually from about 1 to 24 atoms, more usually from about 1 to 12 atoms. Besides carbon atoms, the atoms in the chain may include nitrogen, sulfur, oxygen or the like.

Besides conjugation through a chemical reaction, one can provide for a fused protein by preparing a nucleic acid sequence encoding the ED joined to an amino acid sequence which is immunologically cross-reactive with a peptide of interest. One can synthesize appropriate strands of deoxynucleotides which provide for a fusion protein of the epitope(s) of interest with the ED, where the epitope(s) of interest may be at the N- or C- terminus of the ED, preferably the N-terminus.

The fusion protein may be of any size, usually being note greater than about 500 amino acids, more usually being not greater than about 200 amino acids, and preferably not greater than about 150 amino acids, including the ED sequence.

## Enzyme Acceptors

The enzyme acceptor may be naturally occurring or synthetic. By synthetic is intended the use of recombinant DNA techniques to provide for the desired amino acid sequence. For the most part, the sequence of M15 will be employed as the basic sequence for the enzyme acceptor (EA). Of particular interest is the reduction in the number of available sulfhydryl groups present in the sequence. The EA M15 appears to have 5 cysteine residues available on the surface. Some EAs may have fewer than 5 cysteine residues as a result of substitutions of the cysteine, particularly conservative substitutions, such as G, A, M, S, T, etc.

## Analytes

The analyte may be any member of a specific binding pair, which comprises ligands and receptors where a complementary member of a pair has a high affinity for the other member of the pair, usually at least about $10^{-6}$/mole. The ligands will be at least of about 125D (Dalton) and usually higher, more usually at least about 150D, and may be 500,000D or more. For the most part, the ligands will be less than 200kD, more usually less than 100kD. In many cases, where the ligand is of high molecular weight, greater than 50kD, a fragment of the ligand which is immunologically cross-reactive with the ligand may be employed in the conjugate.

A large number of ligands are listed in U.S. Patent No. 3,996,345, which relevant disclosure is incorporated herein by reference. Ligands for the most part will be drugs, drug metabolites, biologically active molecules, such as steroids, vitamins, proteins, receptors, lymphokines growth factors, etc., industrial pollutants, pesticides and their metabolites, herbicides and their metabolites, flavoring agents, food poisons, components of pathogens, toxins, as well as any other substance of interest.

Receptor analytes may be any protein, nucleic acid or saccharide, which arbitrarily is chosen as the receptor, usually having a cleft or surface concavity where the ligand binds. Receptors for the most part are immunoglobulins, surface membrane proteins, which include T-cell receptors, MHC antigens, blood proteins, such as thyroxine binding globulin, lipoproteins, etc., enzymes, avidin, and the like.

Thus, any compound for which a complementary binding member can be found or prepared may be determined by the subject assays. The analyte need not be a single compound, but an aggregation of compounds such as may be found in microorganisms, such as viruses and bacteria, membrane fragments, or other aggregation or complex organization of molecules.

## Method of Preparation

The subject polypeptide sequences may be prepared by any convenient means. Thus, the sequences may be synthesized on commercially available synthesizers. However, where the sequence is to be greater than about 50 amino acids, the efficiency of synthesis drops, so that other methods may become more attractive. One of the alternative methods is the use of recombinant technology, where single strand deoxyonucleotide sequences are prepared encoding portions of the sequence of interest or sequence complementary thereto. The strands are for the most part overlapping, so that when hybridized and ligated, the resulting double stranded DNA sequence encodes the desired amino acid sequence. The sequence may then be inserted in any convenient expression vector. A large number of expression vectors are commercially available or have been described in the literature. While for the most part prokaryotic hosts will be employed, in some instances eukaryotic hosts will be desirable, particularly where there are fusion proteins and it is desired that the fusion protein be processed. The vector will normally comprise the coding sequence, 5′ in the direction of transcription to the coding sequence, a transcriptional initiation regulatory region or promoter, and 3′ to the coding region in the direction of transcription, a transcription and translation termination regulatory region, so as to provide an expression cassette. Particularly, for transformation into prokaryotes, there will be a replication system which is functional in the host and provides for stable maintenance of the vector. A wide variety of replication systems have been identified and used in prokaryotes, as well as eukaryotes. Also, there will normally be a marker for selection of those host cells which have been transformed with the vector. For the most part, the marker will be resistance to a toxin, e.g., an antibiotic or provide for complementation of an auxotrophic host to provide prototrophy.

Transformation may be achieved by transfection, using a viral vector, protoplast fusion, transformation using calcium precipitated DNA, or other convenient technique. The manners of transformation are conventional and may be found in Maniatis et al, Molecular Cloning: a Laboratory Manual, Coldspring Harbor Laboratory, Coldspring Harbor, NY 1982.

If desired, the sequence may include a signal sequence for secretion of the polypeptide product from the host. A wide variety of signal sequences are available, particularly for eukaryotic organisms. Where a signal sequence is not employed, it will be necesary to lyse the cells in order to extract the desired polypeptide.

The transformed host cells may be grown in an appropriate medium for sufficient time for the desired polypeptide to be formed and the product isolated, the manner depending upon whether the product was secreted or retained in the cytoplasm. Once the product is isolated, it may be purified in conventional ways, by chromatography, electrophoresis, gradient density separation, or the like.

The enzyme acceptor may be prepared in the same way or may be isolated from the host that produces the M15 sequence naturally. The particular manner in which the enzyme acceptor is produced is not critical to this invention.

Once the fragments are obtained, they may be modified as previously described. In the case of the enzyme acceptor, sulfhydryl groups may be capped or otherwise modified as appropriate. A linking group may be introduced onto the polypeptide or the polypeptide may be otherwise modified for reaction with the specific binding pair member portion of the conjugate. The polypeptide may then be combined with the specific binding pair member or analog thereof, and reacted in accordance with the nature of the functional groups and the conditions required for the reaction. For the most part, aqueous media will be used under mild conditions, usually under about 60° C, preferably under about 40° C.

Assay

The protocols for the assay may be varied widely, depending upon whether a manual or automatic system is being employed, the sensitivity of the assay, the speed with which the assay is to be carried out, the nature of the analyte, and the like. The assay may be competitive or non-competitive, again depending upon the nature of the analyte. The various components of the assay may be added sequentially or concomitantly. The sample may be subject to prior preparation or may be used as obtained.

For the most part, the assay medium will be buffered at a pH in the range of about 6 to 8, with a convenient buffer, such as phosphate buffered saline, tris, or the like. The significant factor is that the buffer does not inhibit the enzyme reaction. The ionic strength is not critical. The temperature for the assay will usually be about 20° C, preferably elevated, but below 60° C, preferably below about 40° C. The assays are performed at atmospheric pressure.

The concentration of the enzyme donor conjugate in the assay medium will usually be in the range of about 1nM to about 60nM, more usually about 5nm to 50nM, preferably about 10nm to 25pM. The enzyme acceptor will usually be in substantial molar excess, usually at least about 1.5 molar excess, preferably at least about 5 molar excess. The molar ratios of enzyme donor conjugate to enzyme acceptor conveniently are in the ratio of about 1:30 to 1:80, more usually 1:50 to 1:60. The concentration of the enzyme donor conjugate will usually exceed the highest concentration of the analyte anticipated to be encountered in the sample.

Where ligand is present in the conjugate, the optimal ratio of ED-analyte conjugate and anti-analyte antibody will be determined in the presence of EA so as to span the dynamic range of the assay and also to minimize the background activity. The response of the enzyme-catalyzed rate to analyte concentration in relation to background level is optimized.

The ratio of the concentration of the ED-analyte conjugate and anti-analyte antibody will be such as to substantially achieve minimum enzyme rate under assay conditions in the absence of ligand analyte, while maintaining linearity of the rate varying with analyte concentration over the desired assay range. Usually the concentrations of antibody and conjugate will be within at least about 85%, more usually within at least about 95% of the concentrations necessary to optimize conditions.

Varying amounts of sample can be used, depending upon the concentration of the analyte, the nature of the sample, and the sensitivity of the assay. When the assay is serum, usually the sample will comprise from about 1% to 10% of the volume of the assay medium.

An enzyme substrate is employed that when cleaved by the enzyme results in a change in the amount of light absorbance (optical density) or emission of the assay medium. That is, cleavage of the substrate results in the appearance or disappearance of a colored or fluorescent product. Preferred enzyme substrates include o -nitrophenyl galactoside (ONPG) and chlorophenyl red-$\beta$-galactoside (CPRG). ONPG, CPRG and other comparable enzyme substrates are commercially available. ONPG will generally be used in a concentration of from about 0.5 to 2.0 mg/ml. Other substrates will be used in concentrations to provide a comparable signal to ONPG.

The sample and conjugate may be combined in an appropriate assay medium. The EA may be present or may be added subsequently. The complementary member to the specific binding pair member of the conjugate may be present as the analyte in the sample or may be added as a reagent, normally being present not later than the combining of the ED conjugate and EA. However, one may reverse the order and allow for formation of the enzyme, followed by addition of the complementary specific binding pair member and observe the change in enzyme activity with time. The enzyme substrate may be added any time, but will usually be added after incubation of the various components of the assay.

Usually, one or more readings will be taken after incubation, the interval varying from about 30 sec. to about 20 min., usually from about 1 to 10 min. between the readings. The time for the first reading based on the addition of enzyme substrate will generally be from 30 sec. to about 10 min., more usually within about 5 min. While a single reading may be taken, it will usually be desirable to take more than one reading, so that common errors may be cancelled out.

Desirably, standard solutions will be prepared of known concentrations of analyte to serve as standards for comparison with the sample. In this way, accurate quantitative determinations may be obtained.

The following examples are offered by way of illustration and not the way of limitation.

## EXPERIMENTAL

### Enzyme-Donors

### pI25 Enzyme-Donor

The plasmid pI25 was genetically engineered to place an $\alpha$-donor sequence under regulatory control of a temperature inducible promotor ($\lambda$Pr). In addition, the expressed $\alpha$-donor peptide contains a unique cysteine residue near the C-terminal end. This was accomplished by cleaving the plasmid pUCI3 with BglI and the resultant single-stranded termini were removed by treatment with S1 nuclease. The plasmid was then digested with BaMHI. The approximately 170 bp DNA fragment encoding the $\beta$-galactosidase $\alpha$-gene was then purified by agarose gel electrophoresis. (See U.S. Patent-No. 4,708,929, FIG. 2, in referring to figures in the subsequent description, the figures of the indicated patent are intended).

Plasmid p$\beta$gal2 is a derivative of plasmid pCVQ2 (Queen, 1983, J. Molec. Applied Genetics 2:1) which carries the lac operon under regulatory control of the temperature inducible $\lambda$Pr promotor. To make the $\lambda$ regulatory sequences available for other genetic constructions the plasmid p$\beta$gal2 was modified. Plasmid p$\beta$gal2 was digested with BamHI and SalI and the DNA sequences encoding the lac operon were removed. The DNA fragment containing pBR322 sequences (including amp$^r$ and ori) and $\lambda$CI were isolated by

9

agarose gel electrophoresis. Synthetic DNA linkers containing recognition sequences for BamHI, EcoRI, HindIII, SaII and XbaI were ligated and then cleaved with BamHI and SaII to create shorter multi-linker segments with BamHI and saII cohesive ends. These DNA fragments were ligated to the BamHI/SaII fragment isolated from pβgal2. The resultant plasmid, pI21B contains EcoRI and XbaI recognition sites between the BamHI and SaII of the vector. Plasmid pI21B was digested with BamHI and PvuII. The BamHI/PvuII DNA fragment containing the β-lactamase gene (which confers resistance to ampicillin, amp$^r$), the phage λCl gene (a temperature controlled repressor) and the plasmid origin of replication (ori) was purified by agarose gel electrophoresis. The BglI(-)/BamHI DNA fragment from pUC13 and the BamHI/PvuII DNA fragment from pI21B were ligated using T4 DNA ligase as shown in FIG. 2A. The recombinant plasmid was transformed into JM83, an E . coli bacterial host for growth of the single-stranded phage M13 and its recombinant which encodes the $\overline{\beta\text{-galactosidase}}$ mutant polypeptide M15 (Messing, 1979, Recombinant DNA Technical Bulletin, NIH Publication No. 79-99, 2, No. 2:43-48) and plasmid pI25 was selected, In vivo complementation occurred at 42° C but not at 32° C demonstrating that plasmid pI25 produces a temperature inducible β-galactosidase α-protein.

## H, B, M and P Series Enzyme-Donors

In one series of experiments, to obtain enzyme-donor peptides of the type containing an analyte-coupling domain, various sized α-regions were isolated from pUCl3 (Vieira and Messing, 1982, Gene 19:259-268; Messing, 1983, Methods in Enzymology 101:20-78; Bethesda Research Laboratories, Gaithersburg, MD) digested with HaeII, BglI, MstI or PvuI yielding H-series, B-series, M-series and P-series respectively. The B-, P- and H-series were treated with T4 DNA polymerase and S1 nuclease. The M-series were not treated. Each series of DNA was digested with SacI which is located in the multiple cloning site, and the small DNAs encoding an α-complementing peptide were purified by agarose gel purification, electrophoresed onto DEAE-cellulose paper (Schleicher and Schuell, Keene, NH), eluted and ethanol precipitated as described by the manufacturer.

Plasmid pI41 which carries an E . coli trp promotor (EcoRI-SstI, 120 bp) cloned in the 2.3 kb EcoRI-PvuII fragment of pBR322, was digested with NdeI and treated with DNA polymerase Klenow fragment and dATP and dTTP (PL Biochemicals, Milwaukee, WI). The resultant DNA was digested with SacI and used as a vector to receive the M, B, H and P series of DNAs. Following treatment with T4 DNA ligase, the DNAs were transformed into E . coli strain E9001 (Δlac pro, thi, supE, F′ proAB, lacI$^Q$, Z M15 also referred to as strain 71.18; Messing et al, 1977, Proc. Natl. Acad. Sci. USA 75; 3642-3646). The DNA constructions were sequenced by the method of Maxam and Gilbert (1980, Methods in Enzymology 67:499) and are shown in FIG. 4. Also illustrated (*) are the sites for covalent attachment of an analyte.

The resultant strains encoding α- regions under Trp control in E . coli strain E9001 were for series B, strain MG130 carrying plasmid pI30; for series M, strain MG129 carrying plasmid pI29; and for series H, strain MG131 carrying plasmid pI31.

To improve expression levels of the different cloned α-regions, the α-regions were transferred to new plasmids and placed under control of the λPr operator-promotor. For example, to construct MG141, the gene encoding the DNA sequences of H6 from the H-series was placed under Pr control, by replacement of the Trp promotor for the λPr and λCl genes as described below.

Plasmid pI31, containing H6 under the Trp operator-promotor control was digested with EcoRI and the larger, approximately 2.1 kb fragment was isolated by agarose gel electrophoresis. The λPr and λCl genes were gel purified from the small fragment of an EcoRI digestion of pI25. The 2.1 kb fragment of pI31 was ligated to the small fragment from pI25 in effect replacing the Trp promotor with the λPr and λCl promotor system. This protocol was also repeated with pI30 and pI29 to yield the following plasmids and strains under λPr control for series B, strain MG139 carrying plasmid pI39; for series M, strain MG140 carrying plasmid pI40; and for series H, strain MG141 carrying plasmid pH6. The DNA constructions were sequenced by the method of Maxam and Gilbert, Methods in Enzymology 67:499 (1980), and shown in FIG. 4.

## pI48 Enzyme-Donor

Utilizing the λPr sequence from pI25, a new plasmid was constructed to provide a cysteine residue towards the N-terminal end of the peptide. This new plasmid, pl48, also contained three cysteine residues located near the C-terminal end of the peptide. Plasmid pl25 was digested with BamHI and EcoRI, an approximately 1100 bp fragment was cleaved from the vector and purified by agarose gel electrophoresis. This fragment, contains the λPr sequence which was ligated into the unique BamHI/EcoRI restriction sites of pUCl2, (Messing, 1983, Methods in Enzymology 101: 20-78). This recombinant plasmid was transformed into JM83 cells and found to complement in vivo at 42°C in a manner analogous to the construction of pl25 described supra. The structure of the enzyme-donor pl48 is also shown in FIG. 4, including the positions of amino and sulfhydryl group coupling sites which are utilized according to the present invention for the attachment of analyte.

Enzyme-Donor 3

Enzyme-donor 3 (ED3) was constructed from enzyme-donor 1 (ED1) which was constructed from H6. ED1 was constructed as follows:

Synthesis of DNA fragments was performed on an Applied Biosystems, Inc. (ABI, Foster City, Calif.) Model 380A DNA Synthesizer. Each sequence was entered into the program memory and the machine automatically manufactured the desired single strand of DNA, cleaved each fragment from the controlled pore glass support, and collected the DNA in a vial. DNA samples were treated with 1.5 ml of concentrated $NH_4OH$ for 6-24 hours at 55°C and taken to dryness in a Savant Speed Vac Concentrator.

The dried pellet of each DNA fragment was dissolved in a small quantity of formamide (100-200 $\mu/l$) and purified on a 12% acrylamide gel (BRL Model 50, 34-40 cm, 1.6 mm thickness) and was electrophoresed overnight at 200 volts. The desired band was visualized using Baker-flex silica gel 1B-F (J.T. Baker Chemical Co.) as a fluorescent background. The desired DNA band was cut out of the gel with a razor blade and the DNA electrophoresed from the acrylamide gel fragment in an International Biotechnologies, Inc. (IBI) Model UEA unit following the manufacturer's instructions. The DNA was collected in a small volume of buffer and ethanol precipitated. The fragments were treated with T4 polynucleotide kinase according to the manufacturer's instructions. Complementary DNA strands were combined, heated to 90°C for 2 minutes, and slowly cooled to room temperature. The annealed DNAs were purified by agarose gel electrophoresis to remove unhybridized strands and used in ligation reactions.

The starting plasmid was pl69 which contains the H6 gene under λPr control inserted between restriction sites BamHI and SalI (see FIG. 11). The change from H6 to EDI involved changing both the N-terminus and C-terminus of H6 while leaving the α-domain in between intact. Two aliquots of pl69 were cut with restriction enzymes. The first aliquot was digested with EcoRI and BglI and the small 150 bp fragment was gel purified. The second aliquot of pl69 was digested with BamHI and SalI. This cleaves the plasmid into vector and the α-donor gene region. The vector portion was gel purified.

The new N-terminal coding region of ED1 was a 75 bp DNA fragment synthesized by the Applied Biosystem, Inc. machine (see FIG. 12). The new C-terminal coding region, a 50 bp DNA fragment, was also synthesized (see FIG. 12). The two (2) new DNA fragments were ligated to the small EcoRI-BglI H6 DNA fragment. This mix was cut with BamHI and SalI to yield the new ED gene of about 275 bp. This DNA fragment was gel purified and ligated into the vector Bam-Sal DNA fragment.

After confirming the ED1 sequence, this plasmid (pl81, see FIG. 13) was cut with BamHI and EcoRI which removes the 75 bp ED1 N-terminus. This region was replaced by a newly synthesized fragment of 30 bp (see FIG. 12) substituted into the Bam-EcoRI space

Thus, ED3 is 15 amino acids shorter than ED1 and has a cysteine residue near its N-terminus. ED1 has no cysteine or lysine in its sequence. FIG. 14 depicts the amino acid sequence of ED3.

Enzyme-Donor 3A

The amino acid sequence of enzyme-donor 3A (ED3A) is shown in FIG. 14. The peptide is synthesized on a Beckman (Palo Alto, Calif.) 990BN Peptide Synthesizer. Methods for synthesis are as described by Stewart and Young (Solid Phase Peptide Synthesis, 176pp, Pierce Chemical Co., Rockford, Ill., 1984). General chemicals are from Aldrich (Milwaukee, Wis.). BOC-amino acids are from Peninsula Laboratories (Belmont, Calif.). Side chain protections are Boc-Thr (OBzl), Boc-Glu (OBzl), Boc-Ser (OBzl), Boc-Asp (OBzl), Cys (MeoBzl), Boc-Asn/HOBT, Boc-Arg (TOS) and Boc-His (TOS). Aminomethylpolystyrene solid phase resin beads from Bio-Rad Laboratories (Richmond, Calif.) are esterified to p-hydroxymethyl-phenylacetic acid Boc-Thr (OBZI) with dicyclohexyl carbodiimide as described by Stewart and Young (1984). The synthesis scale used is 1 mmole Boc-Thr attached to the solid phase resin and 3 mmoles of each Boc amino acid. The synthesizer is then programmed to carry out the synthesis. The resultant peptide is cleaved from the resin with anhydrous hydrofluoric acid and extracted with acetic acid. Following hydrogenation, the peptide is purified by preparative reverse phase HPLC using a Waters phenyl column with a 0-80% acetonitrile gradient in water containing 0.1% TFA and 0.1% ethane thio. The partially purified peptide is dialyzed exhaustively into 1 mM $NH_4HCO_3$, 1 mM 2-mercaptoethanol and lyophilyzed. Amino acid analysis of the peptide is shown in Table I.

TABLE I

| AMINO ACID ANALYSIS OF ED3A | | |
|---|---|---|
| AMINO ACID | THEORETICAL | FOUND |
| ASP | 5 | 4.24 |
| THR | 3 | 2.13 |
| SER | 3 | 2.39 |
| GLU | 5 | 5.22 |
| PRO | 3 | 3.33 |
| GLY | 1 | 0.87 |
| ALA | 5 | 5.65 |
| CYS-PE | 1 | 1.10 |
| VAL | 3 | 2.27 |
| MET | 0 | 0 |
| ILE | 1 | 0.48 |
| LEU | 4 | 3.12 |
| TYR | 0 | 0 |
| PHE | 1 | 1.16 |
| HIS | 1 | 1.11 |
| TRP | 2 | 1.61 |
| LYS | 0 | 0 |
| ARG | 5 | 5.00 |
| The molecular weight equals 4942.53 with the average molecular weight of an amino acid being 114.943. | | |

In summary, the polypeptides shown in FIG. 4 provide convenient coupling side chains at varying distances from the required α-complementing sequence. The DNA sequences encoding the peptides made by recombinant methods were determined by the standard Maxam and Gilbert technique, confirming the predicted structures. The amino acid composition of H6 was confirmed by amino acid analysis.


ED Enzyme-Donor Series


A series of enzyme-donors called the ED series was constructed by recombinant DNA techniques. ED3 has already been descsribed. Other members of the series include ED4, ED5, ED7, ED8, ED13, ED14, ED15 and ED17. The amino acid sequences of the ED series of enzyme-donors appear in FIG. 15, A-I.

The gene coding for ED4 was constructed by first synthesizing a DNA fragment on an Applied Biosystems, Inc. Model 380A DNA Synthesizer (as described previously) of the following sequence:

```
            *                50
TGC CCT TCC CAA CAG TTG CGC AGC CTG AAT

TA ACG GGA AGG GTT GTC AAC GCG TCG GAC TTA
___| PvuI

                 60
GGC CTC GAG TCT AGA TCT GCA GGC ATG  (57 mer)

CCG GAG CTC AGA TCT AGA CGT CC  (55 mer)
                            |SphI
```

The "T" marked with an asterisk represents a change from a "C". This fragment was ligated to the BamHI-PvuI piece from plasmid pl81 (ED1) (see FIG. 13). The resultant piece was ligated back into the vector (from ED1-pl81) having removed the BamHI-SphI region. The C to T change creates a cysteine (cys) residue and destroys the PvuI site after ligation. (The sticky ends remain the same for ligation).

The gene coding for EDS was constructed by first synthesizing a DNA fragment of the following sequence:

```
  *                   **  40                      45
  T TGG CGT AAT TGC GAA GAG GCC CGC ACC GAT  (31 mer)

  A ACG GCA TTA ACG CTT CTC CGG GCG TGG C  (29 mer)
  ___|PvuII                             |PvuI
```

The "T" marked with an asterisk represents a change from a "C". The "T" marked with a double asterisk represents a change from an "A". The C to T change destroys the PvuII site. The A to T changes a serine residue to cysteine residue. This fragment was ligated to the BamHI-PvuII piece and PvuI-SalI pieces from plasmid pl82(ED2 or M15)DNA (see FIG. 13). The ligated material was cut with BamHI and SalI and inserted into pl82 with the BaMHI-SalI region removed.

The gene coding for ED7 was constructed by cutting pl83 (ED3) and pl84 (ED4) plasmids (see FIG. 13) with both EcoRI and SalI. The vector from pl83 was gel purified (effectively removing the EcoRI-SalI (α) region. In contrast, the small EcoRI-SalI (α) region from pl84 was gel purified. The pl84 EcoRI-SalI region was then inserted and ligated into the pl83 EcoRI-SalI vector.

The gene coding for EDB was made using site specific mutagenesis in M13mpll phage DNA. A primer was made (sequence GGT AAC GCA AGG GRT TTC CCA GTC). This primer is complementary to the sense strand of the region coding for amino acids 15-22. The desired change was aG to T in codon 20 which changed a Gly to Cys at amino acid 20 in the a region of the M13mpll DNA. This was accomplished by hybridizing the primer to single-stranded M13mpll phage DNA and extending Che primer using DNA polymerase I "Klenow fragment: and T4 DNA ligase overnight at room temperature. This DNA was treated with S1 nuclease to eliminate non-double- stranded DNA and then transformed into JM103 cells. Phage from this transformation were isolated; the DNA was purified and the primer extension and transformation was repeated a total of 3 times. Each repeat enriched for the desired product. Finally, mini-prep analysis was performed on M13 RF DNA from individual placques. The desired base change eliminated the BstNI site. Restriction analysis of mini-prep DNA with BstNI identified candidates. rrom the double-stranded M13 RF DNA carrying the desired change, a BamHI-BglI piece was cut out and exchanged for a BamHI-BglI piece in the plasmid coding for ED2.

The gene coding for ED13 (p193, see FIG. 16) was constructed by first synthesizing (as above) a DNA fragment of the following sequence:

```
                                                          Lys
          ___BamHI                                    change  Eco RI
          |     -3                      0             |  ___|   |  |
     GAT CCC AGC GGC GAT CCC CGG GCA AAA TCG (30 mer)
         GG TCG CCG CTA GGG GCC CAT TTT AGC TTA A (30 mer)
```

This synthesized fragment was substituted into pl82 (ED2) as described above in constructing ED3.

The gene coding for ED14 (p194, see FIG. 16) was constructed by first synthesizing (as above) a DNA fragment of the following sequence:

```
          Lys
        change                         50                      55
         |  ___|
           AAA CCT TCC CAA CAG TTG CGC AGC CTG AAT
       TA TTT GGA AGG GTT GTC AAC GCG TCG GAC TTA
       PvuI
```

```
                              60
       GGC CTC GAG TCT AGA TCT GCA GGC ATG (57 mer)
       CCG GAG CTC AGA TCT AGA CGT GC  (55 mer)
                                      |SphI
```

This synthesized fragment was constructed with the same strategy used for ED4, but resulting in a lysine residue instead of a cysteine substitution.

The gene coding for ED15 (pl95, see FIG. 16) was constructed by first synthesizing (as above) a DNA fragment of the following sequence:

```
                              Lys
                           change 40
       ___          |PvuII     |    |                     |44 PvuI
        T TGG CGT ATT AAA GAA GAG GCC CGC ACC GAT (31 mer)
        A ACC GCA TTA TTT CTT CTC CGG GCG TGG C (29 mer)
```

This fragment was inserted into pl82 (ED2 or M15) in the same way used to construct ED5.

The gene coding for ED17 (pl97, see FIG. 16) is a combination of the ED13 and ED14 genes, constructed in the same way as the gene coding for ED7 was.

The following is a listing of the enzyme acceptors which may be used with the ED series of enzyme donors.

| ENZYME DONOR | ENZYME ACCEPTOR* |
| --- | --- |
| ED3 | M15,EA1,EA14,EA20,EA22 |
| ED4 | M15,EA1,EA14,EA20,EA22 |
| ED5 | M15,EA1,EA14,EA20,EA22 |
| ED7 | M15,EA1,EA14,EA20,EA22 |
| ED8 | M15,EA1,EA14,EA20,EA22 |
| ED13 | M15,EA1,EA14,EA20,EA22 |
| ED14 | M15,EA1,EA14,EA20,EA22 |
| ED15 | M15,EA1,EA14,EA20,EA22 |
| ED17 | M15,EA1,EA14,EA20,EA22 |

*Other enzyme-acceptors have not been tested.

Of the foregoing enzyme-donor and enzyme-acceptor pairs, the ED4 and EA22 combination is a most preferred pair for use in the complementation assays of this invention.

Enzyme-Acceptors

In one group of experiments, a series of in-frame sequence deletions of the $\beta$-galactosidase gene were constructed to prepare a series of enzyme-acceptors according to methods described above. pUCl3 was digested with Pvull (yielding a blunt end) and ligated to an 8 bp synthetic DNA linker containing an Xhol restriction site to create a new plasmid, pUCl3X.

The $\alpha$-region containing the Xhol restriction site was then replaced into the entire lacZ gene, which encodes native $\beta$-galactosidase without disrupting the remainder of the lacZ gene or the background plasmid. The Z gene contains two Bgll sites. The first of these Bgll sites is contained within the $\alpha$-region in pUCl3 downstream from the Pvull site where the Xhol linker was inserted. Thus the $\alpha$-region from pUCl3X was removed from the rest of the plasmid by digesting with BamHI and Bgll and the 170 bp fragment designated B1X.

The remainder of the lacZ gene which encodes $\beta$-galactosidase was obtained from the plasmid p$\beta$gal2 (Queen, 1983, J. Mol. Appl. Genet. 2:1). This plasmid was digested with Bgll and EcoRI and two DNA fragments representing 93% of the Z gene were isolated. The termini of each fragment were different from any other termini used in this construction. The isolated fragments were 2115 bp (hereinafter referred to as B2) and 737 bp [herein after referred to as B3). The EcoRI restriction site in the Z gene is near the C-terminal end of the gene. This terminus must be present when the Z gene containing an Xhol site is constructed.

The mutant Z gene was inserted in pF29. Plasmid pF29 contains a Z gene $\alpha$-region fused to the C-terminal end of the Z gene at the EcoRI site. This $\alpha$-region is controlled by the $\lambda$Pr promotor inserted at a BamHI site. To construct pF29 two intermediate plasmids, pF15 and pF16 were constructed. p$\beta$gal2 was digested with Aval and the cohesive 3′ end filled in using the Klenow fragment and the four dNTPS to create blunt ends. A Sall linker (GGTCGACC) (New England BioLabs, Beverly, MA) was ligated to the linearized plasmid using T4 DNA ligase. The resultant DNA was digested with EcoRI and Sall, and a 300 bp DNA fragment representing the omega ($\omega$) end of the $\beta$-galactosidase Z gene purified by agarose gel electrophoresis. The $\omega$-region was fused to an $\alpha$-region under control of $\lambda$Pr as follows. pUCl2 DNA (Bethesda Research Laboratories, Gaithersburg, MD) was digested with Bgll and blunt ends created by treatment with Klenow fragment and the four dNTPS. EcoRI linkers (GGARTTCC) (New England BioLabs, Beverly, MASS) were ligated to the blunt ends with T4 DNA ligase. The DNA was digested with BamHI and EcoRI and a 180 bp fragment representing the $\alpha$-region of the Z gene was purified by agarose gel electrophoresis. The vector used to accept the $\alpha$- and $\omega$-gene fragments was p$\beta$gal2 digested with BamHI and Sall and purified by agarose gel electrophoresis to remove the lac operon sequences. The vector, $\alpha$-gene and $\omega$-gene fragments were ligated together using T4 DNA ligase. The unique ends of the DNA fragments direct the order in which these fragments were cloned. The product plasmid was designated pF15.

pF15 was further modified by converting the unique Pvull site into the vector Sall site using Sall linkers ligated to the blunt ends created by digesting pF15 with Pvull. This modified pF15 was then digested with BamHI and Sall, and the largest DNA fragment was purified by agarose gel electrophoresis which removes the $\alpha$-$\omega$-gene sequence and a DNA fragement located between the Sall site and the Pvull site. Unmodified pF15 was also digested with BamHI and Sall and the $\alpha$-$\omega$-fragment purified. When the large fragment from the modified pF15 was ligated to the $\alpha$-$\omega$ fragment, the plasmid pF16 was generated.

pF16 is about 1350 base pairs smaller than pF15 and has the effect of moving a unique Ndel site much closer to the Sall site. This maneuver eliminates the unnecessary DNA sequences from being carried through subsequent constructions.

To construct pF29, pF16 was digested with Clal and Ndel and the 1400 bp DNA fragment encoding the $\lambda$Cl, $\lambda$Pr, and the $\alpha$- and $\omega$-regions of $\beta$-galactosidase was purified by agarose gel electrophoresis. pUCl3 was digested with Accl and Ndel and the vector was purified by agarose gel electrophoresis. Since the Accl and Clal restriction sites have identical cohesive ends and the Ndel restriction sites share identical termini, ligation of the DNA insert from pF16 and the pUCl3 vector can occur only in one orientation. Ligation with T4 DNA ligase yielded pF29. pF29 contains one EcoRI site and no Clal sites which was desirable since a second EcoRI and the Clal site would have interfered with the construction of modified plasmids (e.g., pl49 and subsequent analysis of the deletion mutants created from p150 described below).

pF29 was digested with BamHI and EcoRI, the intervening α-donor was removed and this vector was filled-in using B1X plus B2, plus B3 (B1X + B2 + B3). The unique single-stranded end of each piece defines the order in which the pieces can be ligated together. The B1X, B2 and B3 fragments were ligated into the pF29 vector digested with BamHI and EcoRI described above, thus reconstructing a Z gene with an XhoI linker at bp 102 encoding amino acid 34 under λPr control. The resultant plasmid was designated pl49.

To create a method for screening for the creation of viable enzyme-acceptors following digesting with XhoI and Ba131 digestion, a separate α-donor without the XhoI site was inserted into pl49. An FnuDII digestion fragment from pUCl3 containing the lacZ operator, promotor and α-donor was inserted into the SalI site of pl49 which had been filled-in with Klenow fragment. The resultant plasmid was designated pl50. Deletions were created by digesting pl50 with XhoI and then digesting the DNA with Bal 31 exonuclease. After Bal31 treatment, the plasmid was ligated with T4 DNA ligase and transformed into AMA1004 host cells (AMA1004 is galU, galK, strA^r, hsdR^− leuB6, trpC 9830, Δ(lacIPOZ) C29, (Casadaban et al, 1983, Methods in Enzymology, 100:293), and screened on Luria-Bertani plates containing the inducer isopropylthiogalactoside (IPTG) and the chromogenic substrate 5-bromo-4-chloro-3-indoyl-β-D-galactopyranoside (Xgal, Sigma Chemical Co., St. Louis, MO). Colonies that were white at 30°C indicated creation of viable enzyme-acceptors. Colonies were selected and plasmid DNAs prepared. Plasmid DNAs were digested with sail, to remove the α-donor, religated and transformed into AMA1004 host cells. The sequence deletions were confirmed by Maxam and Gilbert sequencing and the enzyme-acceptor proteins purified as described below. The resultant strains are shown in FIG. 5.

Enzyme-acceptors have been constructed utilizing DNA synthesis techniques. For example, enzyme-acceptor 1 (EA1) was constructed from pl49 except that the α-region which contains the XhoI linker was replaced with the following synthesized DNA fragments (5'→3');

| | |
|---|---|
| (1) | CAA CAG TTG CGC AGC CTG AA |
| (2) | AGG CTG CGC AAC TGT TGG GAA GGG CGA TCG |
| (3) | ACC CAA CTT ATT ACC GAT CGC CCT TCC |
| (4) | GTA TAA AGT TGG GTA ACG CCA GGG CCT TCC CA |
| (5) | CAA CGT CGT GAC TGG GAA GGC CCT GGC GTT |
| (6) | GTC ACG ACG TTG TAA AAC GAC GGC CAG TGA ATT CGA GCT CGC CCG GG |
| (7) | GAT CCC CGG GCG AGC TCG AAT TCA CTG GCC GTC GTT TTA |

These fragments encode an in-frame deletion of amino acids 26-43 of the lac Z gene and carry BamHI and BglI sticky ends. These fragments were annealed, purified by gel electrophoresis, treated with BamHI and ligated to B2 plus B3 and the pF29 vector. A positive colony was selected and confirmed by DNA sequence analysis.

In accordance with the aforementioned fragments, analytes may be determined by forming a reaction mixture by combining in a medium (1) the sample; (2) an enzyme-donor polypeptide conjugate; (3) an analyte-binding protein specific for said analyte; and (4) an enzyme-acceptor polypeptide, consisting essentially of a fragment of β-galactosidase. The enzyme-donor polypeptide is characterized by forming with said enzyme-acceptor polypeptide, an active enzyme complex having β-galactosidase activity in the absence of analyte-binding protein binding to said conjugate. The enzyme-donor polypeptide conjugate is further characterized by having substantially as the internal amino acid sequence, amino acids 6-51 of β-galactosidase, having one cysteine, with analyte joined to said one cysteine, and having an N-terminal sequence and a C-terminal sequence, or having substantially as the amino acid sequence, amino acids 7-44 of β-galactosidase, having one cysteine with analyte joined to said one cysteine and having at least an N-terminal sequence. In addition, a substrate capable of reacting with the active enzyme complexes is included in the medium, such that the rate of conversion by the active enzyme complex can be monitored. The enzyme-donor conjugate is further characterized by being capable of competitively binding to the analyte-binding protein which results in inhibiting the formation of the active enzyme complex. After the reaction mixture is formed by combining the above components, the rate of conversion of substrate in the reacation mixture is measured and the amount of analyte is determined by comparing the rate of

16

conversion of substrate in the sample containing medium with the rate of conversion of substrate obtained using a known concentration of analyte.

Of particular interest in the method are enzyme-donor compositions having the one cysteine at amino acid positions 2, 20, 40 or 46, based on the $\beta$-galactosidase numbering as in FIG. 15. Alternatively, the one cysteine may be in the N- or C- terminal sequence, where the N-terminal sequence is up to and including 27 amino acids and the C-terminal sequence is up to and including 17 amino acids.

Of particular interest is an enzyme-donor polypeptide which has an N-terminal sequence as follows:

M D P S G N P Y G I D P T E S S P G N I D P R A S S N,

M D P S G D P R A S S N,

M D P R A S S N, or

C I T D;

and a C-terminal sequence as follows:

A Q P E W G L E S R S A G M P L E,

R S L N G L E S R S A G M P L E, or

R S L N G E L C G V K Y R T D A.

Comparison of Complementation Efficiency

In order to assess complementation efficiency of the enzyme-acceptors prepared as described above representative enzyme-acceptor preparations were compared using H6 as the enzyme-donor.

A microtiter plate format was used comprising a total volume of 200$\mu$l of PM2 buffer (0.5M $Na_2HPO_4$, pH7.0, 1 mM $MgSO_4$, 0.18 mM $MnSO_4$, 1 mM EDTA, 0.02% $NaN_3$, 0.05% Tween 20) containing $2.5 \times 10^{-8}$M of the appropriate enzyme-acceptor preparation and 1.25 mg/ml o -nitrophenol-$\beta$-galactopyranoside substrate. A series of dilutions of H6 (1:20; 1:40; 1:80) were added to initiate complementation. The optical density (414 nm) was measured at 30 and 45 minutes incubation at 37° C. The results are illustrated in Table II.

17

TABLE II

| H6 Dilution | EA23 | EA14 | EA22 | EA24 | EA20 |
|---|---|---|---|---|---|
| | A. $OD_{414}$ After 30 Minutes Incubation at 37°C | | | | |
| 1/20 | .118 | .736 | .708 | .273 | .526 |
| 1/40 | .062 | .351 | .361 | .142 | .277 |
| 1/80 | .030 | .171 | .174 | .071 | .128 |
| | B. $OD_{414}$ After 45 Minutes Incubation at 37°C | | | | |
| 1/20 | .299 | 1.585 | 1.402 | .579 | 1.148 |
| 1/40 | .154 | .776 | .715 | .299 | .610 |
| 1/80 | .068 | .365 | .345 | .147 | .294 |

As demonstrated in Table I, the complementation efficiency of the various enzyme-acceptors varied considerably. The relative complementation efficiencies were:
EA14 = EA22 > EA20 > EA24 > EA23.

EXAMPLE: ENZYME IMMUNOASSAY FOR THYROXINE

This example illustrates an immunoassay for thyroxine using an antibody specific for thyroxine as the analyte-binding protein. The enzyme-donor-antigen utilized is ED4 and the enzyme-acceptor is EA22.

Preparation of Enzyme-Acceptor

The deletion mutant polypeptides of $\beta$-galactosidase was prepared by growing the desired enzyme-acceptor strain in TY broth (1 liter contains Bactotryptone 10 g, yeast extract 5 g, NaCl 5 g and glucose 1 g, pH 7.5). Cells were grown at 42°C. Cells were harvested by centrifugation, washed with breaking buffer (BB)(0.2M Tris®-HCl pH 7.6, 0.2M NaCl, 0.01M Mg acetate, 0.01M 2-mercaptoethanol, 5% glycerol), then pelleted by centrifugation and frozen.

Cell pellets (15 g) were suspended in 40 ml BB. Lysozyme (Sigma Chemical, St. Louis, MO) was added to a final concentration of 0.20 mg/ml and the suspension incubated on ice for 30 minutes. Following incubation, the suspension was frozen in a -70°C alcohol bath and quickly thawed in a 37°C water bath. Care was taken to maintain the temperature of the thawing suspension below 4°C. The vicosity of the lysate was reduced by sonic treatment with a Virsonic cell disruptor (Model 16-850, Virtis Co., Gardiner, NY). Phenylmethylsulfonyl fluoride (PMSF, Sigma Chemical) was added to a final concentration of 0.1 mM, and insoluble material was removed by centrifugation (16,000 X g, 30 minutes). One-tenth volume of a 30% streptomycin sulfate solution was slowly added to the supernatant. After 15 minutes on ice the precipitated nucleic acids were removed by centrifugation at 16,000 X g for 20 minutes. The cleared lysate was brought to 40% saturation with $(NH_4)_2SO_4$ by slowly adding an equal volume of an 80% saturated solution. Following a 2- hour period of stirring at 4°C, precipitated material was collected by centrifugation at 1,000 X g for 30 minutes.

The pellet was redissolved in BB and dialyzed against 1000 volumes of 0.1M $NaH_2PO_4$, pH 7.2, 50 mM NaCl, 1 mM $MgSO_4$, 10 mM 2-mercaptoethanol in water, with one change after 6 hours. The dialyzed enzyme-acceptor extract was applied to a 2.5 X 6 cm column of p-aminophenyl-1-thio-$\beta$-D-galactopyranoside covalently attached to agarose in the same buffer. The column was washed, first with 0.1H $NaPO_4$, pH 7.2, 50 mM NaCl, 10 mM 2-mercaptoethanol, then with 0.1M $NaPO_4$, pH 7.2, 50 mM NaCl, 10 mM 2-mercaptoethanol, and finally with 0.1 M $NaPO_4$, pH 7.2, 50 mM Na borate pH 9.0, 10 mM 2-mercaptoethanol into an equal volume of 2.5M $Tris^R$-HCl pH 7.0. All column operations were performed at 4°C.

The eluted enzyme-acceptor was immediately dialyzed extensively against 0.1M NaH$_2$PO$_4$ pH 7.2, 70 mM NaCl, 1 mM MgSO$_4$ and 10 mM 2-mercaptoethanol. After dialysis glycerol was added to 10% and the extract stored at -20°C. These preparations yielded a single band in the Laemmli discontinuous polyacrylamide gel system (Laemmli, 1970, Nature 227:690).


Preparation of Enzyme-Donors


The various enzyme-donor polypeptides described previously could not be purified from host cells directly. For example, the levels of these peptides found in E . coli strain AMA1004 were insignificant. In contrast, when the plasmids coding for the complementing peptides were transferred to strain E9001 (Δlac-pro, thi, supE.F' proAB, lacI$^Q$, 2 M15 also referred to a 71.18; Messing et al, 1977, Proc. Natl. Acad. Sci. USA 75:3542-3646), active β-galactosidase was formed by in vivo complementation. β-galactosidase was purified and the complementing peptides recovered by denaturation of the enzyme complex with 6M urea.

Cells were grown in Luria-Bertani media supplemented with 0.1% glucose, 50 μg/ml ampicillin, and 0.3 mM IPTG, at 42°C for 16 hours. Cells were harvested by centrifugation. All the following steps were carried out at 4°C unless otherwise noted.

Approximately 40 g of cells from a total culture volume of 12 L were resuspended in 80 ml buffer A (50 mM Tris©, pH 7.5, 50 mM NaCl, 10 mM MgCl$_2$, 10 mM 2-mercaptoethanol). Lysozyme (Sigma Chemical, St. Louis, MO) was added to a final concentration of 0.20 mg/ml and the suspension was frozen in a -70°C alcohol bath and quickly thawed in a 37°C water bath. Care was taken to maintain the temperature of the thawing suspension below 4°C. The viscosity of the lysate was reduced by sonic treatment with a Virsonic cell disruptor (Model 16-850). Phenylmethylsulfonyl fluoride (PMSF, Sigma Chemical) was added to a final concentration of 0.1 mM, and insoluble material was removed by centrifugation at 1,000 X g for 30 minutes. One-tenth volume of a 30% streptomycin sulfate solution was slowly added to the supernatant. After 15 minutes on ice the precipitated nucleic acids were removed by centrifugation at 16,000 X g for 20 minutes. The cleared lysate was brought to 40% saturation with (NH$_4$)$_2$SO$_4$ by slowly adding an equal volume of a 80% saturation solution. Following a 2-hour period of stirring at 4°C precipitated material was collected by centrifugation at 16,000 X g for 30 minutes. The pellet was dissolved in a minimal volume of buffer B (40 mM Tris$^R$, pH 7.5, 0.1M NaCl, 10 mM MgCl$_2$, 10 mM 2-mercaptoethanol) and dialyzed overnight against 200 volumes of the same buffer.

The dialyzed solution was loaded on a 2.5 X 20 cm column packed with 30 ml of DEAE-cellulose (Whatman DE-52), equilibrated with buffer B. The column was washed with 150 ml of buffer B to remove unabsorbed material. Enzyme was eluted with a linear NaCl gradient: 0.01 to 0.50M NaCl in 40 mM Tris$^R$, pH 7.5, 10 mM MgCl$_2$, 10 mM 2-mercaptoethanol. The volume of each buffer component was 75 ml and the flow rate was 0.50 ml/minute. Fractions were assayed for enzyme activity as described. Peak activity appeared at about 0.3M NaCl. Fractions containing enzyme activity were pooled, and the pool was brought to 40% saturation with (NH$_4$)$_2$SO$_4$. After stirring for 2 hours, precipitated material was collected by centrifugation at 12,000 X g for 30 minutes. The pellet was dissolved in a minimal volume of buffer B, then loaded on a 1.0 X 120 cm column packed with Bio-Gel A-1.5 m (bed volume 86 ml, Bio-Rad Laboratories, Richmond, CA). The column was developed with buffer B at a rate of 0.10 ml/minute. Fractions were assayed for enzyme activity, and fractions containing peak activity pooled. An equal volume of 100% saturated (NH$_4$)$_2$SO$_4$ solution was slowly added. After 2 hours on ice, precipitated material was collect by centrifugation at 12,000 X g for 30 minutes.

The pellet was dissolved in a minimal volume of 50 mM KH$_2$PO$_4$, pH 7.3, 1 mM EDTA. 0.496 g of solid electrophoresis purity urea (Bio-Rad, Richmond, CA) per ml of solution was slowly added, bringing the final urea concentration of the pool to 6.0M. The pool was kept on ice until no enzyme activity was visible for five minutes after addition of substrate. The denatured enzyme pool was then loaded on a 1.0 X 120 cm column packed with Sephadex G-75 (bed volume 84 ml, Pharmacia Fine Chemicals, Piscataway, NJ). The column was developed with 6.0M urea, 50 mM Tris$^R$, pB 7.6, 0.15M NaCl, 1 mM EDTA, at a flow rate of 0.10 ml minute. Fractions were assayed for complementation activity with M15. Fractions containing complementation activity were pooled. The fraction pool was dialyzed 3 times against 4 L of 1 mM NH$_4$HCO$_3$ and lyophilized.


Thyroxine Immunoassay

The enzyme-donor conjugate of m-maleimidebenzoyl-L-thyroxine-ED$_4$ was prepared as follows:

L-thyroxine, free acid (680 mg) was covered with anhydrous methyl alcohol (6.0 ml) and the solution saturated with a vigorous stream of dry hydrogen chloride. After cooling, the saturation procedure was repeated and the solvent removed under reduced pressure. The resultant crystalline precipitate was filtered off, washed with absolute ethyl alcohol, then diethyl ether, and finally dried. The dried thyroxine methyl ester hydrochloride was dissolved in 50% aqueous ethyl alcohol and the solution treated with 2N sodium hydroxide (one equivalent). A copious white precipitate formed immediately and additional water was added to complete the precipitation. After allowing the precipitated L-thyroxine methyl ester free base to stand in the cold for one hour, the product was recovered by centrifugation and dried in vacuo. L-Thyroxine methyl ester free base (10 mg) and 5mg m-maleimidobenzoyl-b-hydroxysuccinimide ester (MBSE), (Pierce Chemical Co., Rockford, ILL) were dissolved in 1.0 ml of anhydrous tetrahydrofuran followed by the addition of 10 mg of anhydrous powdered sodium carbonate. The mixture was refluxed for 30 minutes. Examination of the reaction mixture by thin-layer chromatography (TLC) using silica gel G using Si$\alpha$ 250P TLC plates 50 X 20 cm (Baker, Phillipsburg, NJ) containing a fluorescent indicator and ethyl acetate as the solvent system showed the reaction to be approximately 70% complete. The product of L-thyroxine methyl ester free base and MBSE, m-maleimide-benzoyl-L-thyroxine (MBTM) was purified by a silica gel column using chloroform:methanol mixtures as eluting solvents. The isolated pale yellow powder of MBTM was approximately 80% pure as assessed by TLC and had an R$_f$ completely distinct from either MBSE or L-thyroxine methyl ester. The MBTM gave the characteristic orange color for thyroxine upon irradiation with short wave length UV light on silica gel G containing a fluorescent indicator, was ninhydrin negative and the presence of the maleimide group confirmed by its ability to react with cysteines using 5,5'-dithiobis-(2-nitrobenzoic acid) (Sigma Chemical, St. Louis, MO).

ED$_4$ enzyme-donor polypeptide (10 $\mu$g) was dissolved in 0.15 ml of 0.1M NaPO$_4$ pH 7.0 to the above stirred solution were added two 5$\mu$l aliquots of m-maleimidibenzoyl-L-thyroxine methyl ester 0.3 mg in 1.0 ml tetrahydrofuran. After stirring for 1 hour at room temperature the reaction mixture was purified on a Bio-Gel P-2 column (Bio-Rad, Richmond, CA) 0.6 X 16.0 cm, eluting with 0.1M sodium borate buffer, pH 9.0. Ten drop fractions were collected. Aliquots of each fraction were assayed for complementation activity in the presence of the EA23 dimer and o-nitrophenyl-$\beta$-D-galactopyranoside. Fractions 10 and 11 contained the highest complementation activity and were pooled.

This example illustrates an immunoassay for thyroxine as analyte using H6-thyroxine conjugate as enzyme-donor, EA23 as enzyme-acceptor, anti-thyroxine antibody and a series of concentrations of thyroxine.

Reagents for the assay were prepared as follows:

L-thyroxine standard: 2.6 mg L-thyroxine (Sigma Chemical, St. Louis, MO) was dissolved in 200 $\mu$l absolute ethanol and 800 $\mu$l 0.15M NaHCO$_3$ added and the mixture stored at 25°C. Two fold dilutions of thyroxine were prepared in ethanol: 0.15M NaHCO$_3$ (1:4).

L-thyroxine antibody: Antisera to thyroxine (T4) was purchased from Western Chemical Research Corp., Denver, CO. Several lots were tested for titer and an equilibrium constant determined in a radio-immunoassay with IgM Sorb (Enzyme Center, Malden, MA). Lots varied with titers of 1:100 to 1:8000. Equilibrium constants varied from 4.5X10$^8$L/mole to 1X10$^{10}$L/mole. Lot #A420, titer 1:8000 (zero binding-67%) and Keq = 2X10$^{10}$L/mole was used.

EA23 acceptor-enzyme: 6.3X10$^{-7}$M in storage buffer. Substrate: o-nitrophenyl-$\beta$-D-galactopyranoside (ONPG) was dissolved in 2.5 X Z buffer to a final concentration 10 mg/ml solution.

The assay was performed in microtiter plates (Dynatech Cat. #001-012-9200 American Scientific Products, Sunnyvale, CA) and read on a Titertak Multiscan microtiter plate reader fitted with a 414 nm filter (Flow Laboratories, Rockville, MO). To each well was added 100 $\mu$l of PM2 buffer containing 0.05% Tween 20 (polyoxyethylene sorbitan monolaurate) (Sigma Chemical Co., St. Louis, MO). To each well was added sequentially 2.5 $\mu$l H6-thyroxine conjugate, 2.5 $\mu$l of anti-thyroxine antibody, 2.5 $\mu$l of the thyroxine standards and 40 $\mu$l of EA23. Results are illustrated in Table III.

EP 0 419 081 A2

## TABLE III

| ENZYME IMMUNOASSAY FOR THYROXINE | | | | | |
|---|---|---|---|---|---|
| Well | ED$_4$-T4[a] ($\mu$l) | Antibody ($\mu$l) | Thyroxine ($\mu$g/$\mu$l) | EA2[b] ($\mu$l) | OD$_{415}$ |
| 1 | -- | -- | -- | 40 | 0.002 |
| 2 | 2.5 | -- | -- | -- | 0.001 |
| 3 | 2.5 | -- | -- | 40 | 0.595 |
| 4 | 2.5 | 2.5 | -- | 40 | 0.300 |
| 5 | 2.5 | 2.5 | 6.25 | 40 | 0.312 |
| 6 | 2.5 | 2.5 | 12.5 | 40 | 0.320 |
| 7 | 2.5 | 2.5 | 25 | 40 | 0.364 |

(a) ED$_4$-T4 designates the m-maleimide-benzoyl-L-Thyroxine-ED$_4$ conjugate.
(b) EA23 designates the enzyme-acceptor polypeptide.

## N-Terminal Fusion

Plasmid pBR322 containing the entire genome of HBV inserted in the unique EcoRI site was cleaved with HincII. Fragment B (Sninsky et al, 1979, supra) was cloned into the unique HincII site of pUCI3 (Messing, 1983, supra). From this clone a BamHI-AhaIII fragment containing most of the HBV-SAg gene was inserted into pUCI3 digested with BamHI and SalI. This recombinant DNA plasmid 122 was transformed into the JM83 strain of E . coli and light blue colonies, indicating in vivo complementation by a HBV-SAg enzyme-donor, on Xgal plates selected. This clone, MG122, was found to contain HBV-SAg by cross-reaction in the Abbott Auszyme II(1) test (Abbott Laboratories, Chicago, ILL). This HBV-SAg α-donor fusion can be transferred to another expression vector to yield large quantities of fusion product.

## C-Terminal Fusion

For example, the Hepatitis B Virus surface antigen (HBV-SAg) could be cloned at the carboxy terminus of an enzyme-donor polypeptide. One protocol that could be utilized is briefly outlined below as an illustrative example.

A 1.2 kb FnUDII fragment is isolated from a clone of the entire HBV genome and inserted in pBR322. A PvuI partial digest of pI25, treated with S1 nuclease and calf intestinal phosphatase, is then agarose gel purified to isolate full length linear molecules. Following ligation of the FnuDII fragment to the linear DNA of pI25, the DNA is transformed into an E . coli host (e.g., JM83). Ampicillin resistant colonies, white at 30° C and blue at 42° C on Xgal plates, are then selected and screened for production on HBV-SAg (e.g., by the Abbott Auszyme II test). The fusion proteins are then purified by standard ion-exchange and affinity column techniques assaying for complementation.

## Enzyme Immunoassay For HBV-SAg

An immunoassay to measure the presence or quantity of HBV-SAg in a sample can be prepared by competing unknown HBV-SAg fusion protein for homologous antibody. The amount of free α-HBV-SAg protein available to complement EA23 producing active β-galactosidase will be inversely proportional to the amount of unknown free HBV-SAg measured.

## EXAMPLE: HEPATITIS B VIRUS

21

## CORE ANTIGEN ASSAY

The Hepatitis B virus (HBV) genome DHA was cleaved with restriction enzymes BamHI and EcoRI to produce 2 large DNA fragments. One of these large fragments carries the core gene which encodes the core antigen (HBV-CAg). This fragment was inserted into the multiple cloning site of M13 mp10 RF DNA. After selecting and screening for an M13 phage which carries this HBV insert, a small preparation of phage was purified. Single-stranded DNA whcih carries the (-) polarity strand (opposite polarity to messenger RNA) of the core gene was isolated from the phage.

Like most genes, the core gene begins with an ATG codon. Since the expression vector in which the core gene was cloned already supplied an ATG codon, it was necessary to obtain a DNA fragment which began at the second core codon. This was accomplished by synthesizing twelve pair strand (12) single oligomer which represents the (+) strand (the same polarity as messenger RNA) of codons 2-5 of the core gene (GACATTGACCCT). This oligomer was hybridized to the single-stranded M13 phage DNA and extended in vitro by E . coli DNA polymerase I (Klenow fragment). This preparation was digested with HincII, which cleaved the HBV DNA outside of the core gene 3′ to the translation termination codon. Thereafter, nuclease S1 was used to digest the single-stranded DNA 5′ to the second codon of the core gene. This leaves a 686 base pair fragment and many smaller double-stranded fragments of various lengths. The 686 base pair fragment was purified by agarose gel electrophoresis. The plasmid expression vector used carried a λPr promotor and ATG start codon next to a BamHI restriction site. The vector was digested with BamHI and treated with nuclease 51 to render the vector blunt-ended.

The blunt-ended expression vector and the core gene fragment were then ligated together, using T4 DNA ligase, and transformed into competent bacteria. The resultant colonies were screened, and a plasmid identified, carrying the core gene inserted in the proper orientation. Colonies were tested for the presence of core antigen protein in the cell lysate by the Abbott Core Antigen ELISA test (Abbott Laboratories). A strongly immunoreactive positive clone, designated MG152 containing plasmid pl52, was selected and the DNA sequence confirmed by Maxam and Gilbert DNA sequencing. Core antigen is purified and used to produce antibody.

Since none of the restriction sites at the amino terminal end of the α-region of pF29 were compatible for fusion of the core gene to the α-region, it was necessary to construct a second plasmid with different restriction sites in the multiple-cloning region at the amino terminal end of the α-gene. pUCl3 was digested with EcoRI and the cohesive ends filled-in with DNA polymerase large fragment (Klenow fragment) plus all four dNTPS. A PvuII 8 bp (GCAGCTGC) linker DNA was ligated into this site. This modified plasmid was digested with BamHI and PvuII and the N-terminus of the α-piece with the addition of the PvuII linker in the multiple-cloning site isolated. pF29 plasmid DNA was also digested with BamHI and PvuI and the pF29 α-region was removed and replaced with α-region containing the new sequence in the multiple-cloning region of the N-terminus of the α-region. This new plasmid was designated pl54.

To construct a core-α fusion protein, the core gene from pl52 under λPr control was inserted into the multiple-cloning site of the α-gene of pl54. pl54 DNA was digested with restriction enzymes BclI and AvaI. The intervening DNA fragment created by this cleavage carries most of the λCI gene and the λPr promotor plus the core gene but without the four 3′-terminal codons of the core gene. This DNA fragment was purified by agarose gel electrophoresis. Plasmid pl54 was digested with restriction enzymes BclI and XmaI and the intervening piece was removed and replaced by the BclI-AvaI DNA fragment from pl52 (XmaI and AvaI have compatible cohesive ends). Thus, the core gene under λPr control minus the four terminal 3′ codons was inserted into the multiple-cloning site of the α-region in pl54 creating an in-frame gene fusion expressing an HBV core antigen-α fusion peptide. This new core-α expressing plasmid is referred to as plasmid pl57. The fusion peptide is purified and used with antibody to construct an immunoassay for Hepatitis core antigen in a procedure analogous to that outlined for HBV-SAg.

## EXAMPLE: ASSAY FOR BIOTIN

This example illustrates a competitive binding assay for biotin utilizing the glycoprotein avidin as the analyte-binding protein.

Avidin (MW = 67,000 daltons) binds biotin (MW = 244 daltons) with an association constant of $10^{15}$ L/mole. Biotin was bound to the lysine at position and the N-terminal $\alpha$-amino group of H6. Avidin in solution was used as the analyte binding protein to determine whether the avidin coupled to the enzyme-donor inhibited complementation with EA23.

Coupling of biotin to the enzyme-donor H6 was performed as follows. Lyophilized H6, prepared as described above, was dissolved in 0.15 ml of 0.1M Na phosphate, pH 7.5 and stirred at room temperature. Two 5 $\mu$l aliquots of N-hydroxysuccinimidobiotin (Sigma Chemical, St. Louis, MO) at 10mg/ml in N,N-dimethylformamide (DMF) were added. After one hour at room temperature, the solution was centrifuged and the supernatant applied to a Bio-Gel P-2 (0.6X16 cm) sizing column (BioRad Labs, Richmond, CA) equilibrated with 0.1M Na borate pH 9.0 and eluted with the same buffer. Ten drop fractions were collected and fractions containing the biotinyl-H6 conjugate (i.e., complementation activity) were pooled.

In a preliminary experiment, a titration was performed to determine the concentration of avidin required to inhibit complementation. PM2 Buffer, a biotinylated-H6, avidin, EA23 and substrate o-nitrophenyl-$\beta$-D-galactopyranoside were added to microtiter plate wells. After 15 minutes at 37° C, the optical density at 414 nm ($OD_{414}$) was determined. Table IV shows the results. This data demonstrates that 0.5 $\mu$g avidin (7.5 X $10^{-12}$ moles) inhibits 75% of the complementation reaction.

TABLE IV

| INHIBITION OF COMPLEMENTATION BY BINDING TO AVIDIN[a] | | |
|---|---|---|
| Well | Avidin ($\mu$g) | $OD_{414}$ |
| 1 | 0 | 0.545 |
| 2 | 0.1 | 0.499 |
| 3 | 0.2 | 0.438 |
| 4 | 0.3 | 0.370 |
| 5 | 0.5 | 0.133 |
| 6 | 1.0 | 0.123 |

(a) 2.5 $\mu$l of Biotinylated-H6, prepared as described; 20 $\mu$l EA23 (3.6X10$^7$M); and 100 $\mu$l substrate o-nitrophenyl-$\beta$-D-galactopyranoside (ONPG) (10 mg/ml) were used/well. Sufficient PM2 Buffer was added to e ch well to bring the final volume to 200 $\mu$l.

A competitive binding assay for biotin was performed as described for the preliminary experiment, except that varying concentrations of free D-biotin (Sigma Chemical, St. Louis, MO) were added to generate a competitive binding curve. Thus, each well contained 5 $\mu$l EA23 (3.6X10$^{-7}$M); 100 $\mu$l ONPG (10 $\mu$g/ml) with sufficient PM2 Buffer to bring the total volume to about 200 $\mu$l. The optical density (414 nm) was measurea after 15 minutes. As demonstrated, this assay system provides a good assay for biotin over the range of 1 to 8 mg or 4-32X10$^{-12}$M biotin. The avidin-biotin system ($k_\alpha$ = 2X10$^{15}$ L/mole) has sufficient affinity to control complementation ($k_\alpha$ = 1-2X10$^5$ L/mole) within a 15 minute assay.

EXAMPLE: HETEROGENEOUS COMPLEMENTATION ASSAY FOR BIOTIN

This example illustrates a heterogenous assay system for biotin utilizing avidin as the specific analyte-binding protein. The enzyme-acceptor is EA23, and the enzyme-donor is CNBr2 coupled to biotin (hereinafter, CNBr2-biotin conjugate).

CNBr2-biotin conjugate was synthesized as follows: 900 $\mu$g of lyophilized CNBr2 polypeptide was dissolved in 300 $\mu$l of 0.1M sodium phosphate buffer, pH 7.5. A 200 $\mu$l aliquot of N,N-dimethylformamide (DMF) containing 2.1 mg of [N-hydroxy-(D-biotin succinimide ester, or N-hydroxysuccinimidobiotin) suc-

23

cinimide activated biotin (Sigma Chemical Co., St. Louis, MO)] was added in 20 μl aliquots with stirring at room temperature. After 2 hours, the reaction mixture was chromatographed on a Biogel P-2 column (1.5X48 cm) using 0.1M sodium borate buffer, pH 9.0. The fractions containing the CNBr2-biotin conjugate were identified by the complementation reaction with EA23.

Avidin immobilized agarose (avidin-agarose, Sigma Chemical Co., St. Louis, MO) 17.5 units per μl suspension, where 1 unit binds 1 μl/g of biotin) stock was diluted in a low gelling temperature agarose suspension (6 mg/ml) to give the desired level of avidin-agarose.

### Inhibition of CNBr2-Biotin Complementation Activity by Avidin-Agarose

20 μl of CNBr2-biotin conjugate stock (5 X $10^{-7}$M), 90 μl of PM2 Buffer and 20 μl of avidin-agarose of various dilutions were mixed well in Eppendorf vials and incubated at room temperature for 10 minutes. The vials were then centrifuged for 5 minutes and 100 μl of the supernatant was removed from each vial into microtiter wells, each containing 10 μl EA23 stock (1.5 X $10^{-6}$M) and incubated at 37°C for 15 minutes. The substrate ONPG (100 μl of 10 mg/ml) was then added and the absorption of each well at 414 nm was measured after 30 minutes at 37°C to obtain a tighter value by graphing the results.

### Competition of Biotin with CNBr2-Biotin Conjugate For Immobilized Avidin

Using the titer value determined above, the biotin dose response curve is obtained as follows. 20 μl of avidin-agarose suspension (total 0.35 units), and 90 μl of PM2 Buffer containing various levels of biotin were mixed well in Eppendorf vials and incubated at room temperature for 10 minutes. Then 20 μl of CNBr2-biotin conjugate stock (5 X $10^{-7}$M) was added, mixed well and incubated at room temperature for 10 minutes. The vials were then centrifuged for 5 minutes and 100 μl of the supernatant was removed from each vial into microtiter wells, each containing 10 μl EA23 stock (1.5 X $10^{-6}$M) and incubated at 37°C for 15 minutes. Substrate ONPG (100 μl of 10 mg/ml) was added and the absorption of each well at 414 nm was measured after 30 minutes incubation at 37°C. The dose response curve was graphed. Such a curve can be used to quantitate the amount of biotin in an unknown sample.

### EXAMPLE: ENZYME IMMUNOASSAY FOR DIGOXIN

This example illustrates an enzyme immunoassay where the analyte is the cardiotonic digitalis glycoside digoxin. The analyte-binding protein is an antibody specific for digoxin. The example further demonstrates that the mechanism of action of the assay is not analogous to the steric hinderance enzyme immunoassay using β-galactosidase described by Castro and Monji (1981, Methods in Enzymology 73:523-42).

### Preparation of Digoxin-ED₄ Conjugate

A urethane derivative of digoxigenin specifically 3-O-[m-maleimidophenylcarbamyl] digoxigenin [hereinafter termed "digoxin-malemide adduct"] was prepared as follows:

To a dry 10 ml round bottom flask equipped with a magnetic stirring device, an argon inlet, and a reflux condenser, was added 3-carboxylphenylmaleimide (67 mg or 0.307 mmole), dry benzene (3 ml), and dry triethylamine (0.043 ml or 0.307 mmole). The mixture was refluxed for 30 minutes. An infrared spectra analysis (IR) of an aliquot showed conversion to carbonyl azide (2150 $cm^{-1}$). Digoxigenin (80 mg or 0.205 mmole) and dry tetrahydrofuran (2 ml) were then added to the reaction mixture. After 3.5 hours of refluxing, the reaction mixture was diluted with ethyl acetate (100 ml), washed once with 50 ml cold 1% aqueous NaOH, and once with 50 ml saturated aqueous NaHCO₃. The organic layer was then dried over anhydrous MgSO₄, filtered, and the solvent removed by rotary evaporation. The residue was dissolved in approximately 1-2 ml acetone and applied to two preparative thin layer chromatography (TLC) plates (1500 micron silica gel Analtech uniplate, Analtech, Newarl, DE). When the acetone evaporated, the plates were eluted with 80/20 ethyl acetate/benzene. Unreacted digoxigenin was removed from the plate by washing it 3 times with 30 ml of ethyl acetate. This process was repeated for the next two spots above digoxigenin. This purification afforded digoxigenin (26 mg), the desired product digoxin-maleimide adduct (31 mg or 37% yield based on unreacted starting material), and $^{3-}$0-(m-maleimido-phenylcarbamyl)digoxigenin (28 mg or 33% yield based on reacted starting material).

Thin layer chromatography was performed in 2.5% MeOH-$CH_2Cl_2$ to ascertain the purity of the digoxin-maleimide adduct. If further purification is desired, this is best accomplished by preparative TLC with 3% MeOH/$CH_2Cl_2$ (2 elutions). The digoxin-maleimide adduct had the following spectral characteristics: Infrared (nujol mull): 3490, 3350, 1805, 1760, 1725, 1700, 1615, 1550, 1460, 1305, 1240, 1160, 960, 935, 905, 880, 840, 810, 790, 710 cm$^{-1}$. (NMR, Nuclear Magnetic resonance acetone $d_6$): 0.8 (3 H,s), 3.38 (1 H, brs), 3.40 (1 H, q, J = 4.78 Hz), 4.84 (2 H, t, J = 1.5 Hz), 5.00 (1 H, m), 5.78 (1 H, t, J = 1.5 Hz), 6.98 (s, 2 HO), 6.8-7.7 (4 H, m), 8.75 (1 H, br s). Mass spectrum (CDI-$NH_3$): 622 (M + $NH_4$ +) 605 (M + H)30, 587 (M + H + -$H_2$), 391, 373, 355, 337, 214, 191, 189.

The digoxin-maleimide adduct was then further purified on a RP-8 SynChropak 250 X 10 mm I.D. (SynChrom, Inc., Linden, ID) using a Beckman Model 332 high performance liquid chromatography system (Beckman Instruments, Inc., Palo Alto, CA). Gradient elution was performed from 0-80% acetonitrile in $H_2O$ over 60 minutes at a 1.5 ml/minute flow rate. The digoxin-maleimide adduct was pooled and lyophilized.

The purified digoxin-maleimide adduct was then coupled to the enzyme-donor $ED_4$, prepared as described supra, to form digoxin-$ED_4$, an enzyme-donor analyte conjugate. $ED_4$ (1.5 mg) was dissolved in 240 $\mu$l acetonitrile-50 mM sodium phosphate (3:2) at pH 6.0. Digoxin-maleimide adduct (1.0 mg) was added directly to the reaction mixture which was maintained at 37°C for two hours. Upon completion of the coupling reaction, 60 $\mu$l aliquots of the mixture were injected onto a Bondapak® Phenyl column 10 X 30 cm (Waters Associates, Milford, MA). The column was developed with a 60 minute gradient 0-80% acetonitrile in $H_2O$, 0.1% trifluoroacetic acid. Samples containing enzyme-donor activity were pooled.

Immunoassay For Digoxin

In enzyme immunoassay systems prepared according to the methods of the present invention, varying combinations of concentrations of enzyme-acceptor and enzyme-donor conjugate (i.e., enzyme-donor coupled to analyte) can be used to produce a given $\beta$-galactosidase concentration via the complementation process. The law of mass action requires that at relatively high concentrations of enzyme-acceptor, the inhibitory influence of the antibody on the complementation process is mitigated. This is evidenced by flat or absent dose-response characteristics with varying concentrations of analyte, e.g., digoxin. Conversely, at relatively high concentrations of enzyme-donor conjugate (compared to antibody), the inhibitory influence of the antibody on the complementation process is also lost. The latter situation is also evidenced by flat or absent dose-response characteristics and an elevated background.

This example illustrates that just as in conventional enzyme immunoassays, the relative concentrations of enzyme-acceptor, enzyme-donor and specific antibody must be defined to produce an assay with dose-response characteristics having suitable precision (slope) and sensitivity for use in a diagnostic assay for analyte.

In one series of experiments using a microtiter format, the sensitivity of the system was determined using different combinations of digoxin-$ED_4$ enzyme-donor and EA23 enzyme-acceptor concentrations.

Assays were performed by adding four sequential additions of 50 $\mu$l each, digoxin (analyte), enzyme-donor H6 digoxin conjugate, antibody specific for digoxin (anti-digoxin) and solution containing both enzyme-acceptor (EA23) and o-nitrophenyl-$\beta$-D-galactopyranoside (ONPG) 5 mg/l as substrate. All dilutions were performed in PM2 Buffer [0.5M $Na_2HPO_4$, 1 mM $MgSO_4$, 1 mM EDTA, 0.02% $NaN_3$ and 0.05% Tween 20 (polyoxyethylene sorbitan monolaurate, Sigma Chemical Co., St. Louis, MO). The concentrations of the digoxin analyte were: 0, 1, 10, 100, 200, 500 and 1000 ng/ml. Antibody specific for digoxin was obtained by injection of digoxin conjugate into rabbits as follows: Primary intramuscular injections were made using 50 $\mu$l of conjugate in a total volume of 2.0 ml complete Freund's adjuvant. Boosters (intramuscular) were administered at 4-week intervals with 25 $\mu$g of conjugate in a volume of 1.0 ml complete Freund's adjuvant. 50 ml bleeds were collected every two weeks starting 90 days following primary injection. Collections were made by phlebotomy of the medial artery or lancing of the marginal ear veins. Blood was allowed to coagulate and 25 ml serum/50 ml blood recovered as supernatant following 30 minutes centrifugation at 1000 X g. The results were graphed. A comparison of the dose-response curves in

EP 0 419 081 A2

showed that selective reduction of the concentration of either enzyme-acceptor or enzyme-donor conjugate produces a steeper, and hence more sensitive dose-response curve.

## Mechanism of Digoxin Immunoassay

In order to determine whether reacting the anti-digoxin antibody with the enzyme-donor digoxin conjugate was interfering with the complementation process rather than with conversion of substrate by polymerized $\beta$-galactosidase enzyme, the complementation process was allowed to proceed to completion prior to addition of antibody in one series of experiments.

The protocol of the experiments was as follows: 300 $\mu$l of PM2 Buffer and the digoxin-H6 conjugate were reacted for 60 minutes with 150 $\mu$l of the enzyme-acceptor EA23 (4.1 X $10^{-6}$ M). This permitted the complementation to proceed to completion. An aliquot (125 $\mu$l) of the above reaction mixture was removed and added to an aliquot (50 $\mu$l) of rabbit anti-digoxin antibody (diluted 1:100 with PM2 Buffer). The reaction mixture was then incubated for 30 minutes. At the end of this time period, the ONPG substrate (final concentration 1 mg/ml) was added and the reaction mixture incubated at 37° C. The optical density of the reaction mixture was determined at 7 and 16 minutes following incubation at 37° C. Control tubes were treated similarly except that 50 $\mu$l of either normal rabbit serum diluted 1:100 with (PM2 Buffer) or PM2 Buffer was added instead of rabbit anti-digoxin antiserum. Results are illustrated in Table V.

### TABLE V

| | Optical Density Incubation Time | |
|---|---|---|
| Sample | 7 Minutes | 16 Minutes |
| Anti-digoxin[a] | .475 | .947 |
| NRS[b] | .466 | .954 |
| PM2[c] | .457 | .936 |
| Substrate Blank | .050 | .057 |

(a) Anti-digoxin designates rabbit 539 (50 $\mu$l, 1:100 dilution in PM2 Buffer).
(b) NRS designates normal rabbit serum (50 $\mu$l 1:100 dilution in PM2 Buffer).
(c) PM2 Buffer: 0.5 M $Na_2HPO_4$, pH 7.0, 1 mM $MgSO_4$, 0,18 mM $MnSO_4$, 1 mM EDTA, 0.02% $NaN_3$, 0.05% Tween 20).

As demonstrated in Table V, antibody did not inhibit conversion of substrate by the previously polymerized $\beta$-galactosidase (complete complementation of Digoxin-$ED_4$ and EA23 enzyme-acceptor). Thus, the decreased substrate conversion observed using the enzyme assay is the result of antibody-inhibited complementation, nor reduced enzyme substrate conversion. Therefore, the mechanism of action of the assay of the present invention is not analogous to the steric hinderance enzyme immunoassay using $\beta$-galactosidase described by Castro and Monji (1981, Methods in Enzymology 73:523-542).

## Effect of Anti-Digoxin Antibody on Complementation Using a Variety of Enzyme-Acceptors

In one series of experiments, the inhibitory effect of specific antibody against digoxin was determined using three enzyme-acceptors and the enzyme- donor digoxin-H6 conjugate prepared as described above.

The reaction mixture was prepared as follows: 50 $\mu$l PM2 Buffer, 50 $\mu$l of the appropriate dilution (1:20, 1:40, 1:80) of digoxin-$ED_4$ conjugate in PM2 Buffer; 50 $\mu$l of the appropriate antibody (i.e., either anti-digoxin antibody to normal rabbit serum) and 50 $\mu$l of the appropriate mixture of enzyme-acceptor (1 X $10^{-7}$ EA14, EA20 or EA22) and substrate o-nitrophenol-$\beta$-D-galactopyranoside (ONPG) (5 mg/ml) were added to a microtiter plate. The plates were then incubated at 37°C for specified time periods. The optical density at 414 nm was determined at 5 minute intervals for 45 minutes.

The following table indicates α-region enzyme : acceptor sequences.

```
    *                        10                      20
    M  D  P  R  A  S  S  N  S  L  A  V  V  L  Q  R  R  D  W  E  N  P  G  V  T  E

                     30                      40
    L  N  R  L  A  A  H  P  P  F  A  S  W  R  N  S  E  E  S  R  T  D  R  P  S  Q

    50                      60
    Q  L  R  S  L  N  G  E  L  R  F
```

|      | Amino Acid Deletions |
|------|----------------------|
| M15  | 11 - 41              |
| M112 | 23 - 31              |
| EA5  | 35 - 52              |
| EA11 | 35 - 54              |
| EA14 | 30 - 37              |
| EA17 | 21 - 53              |
| EA18 | 13 - 45              |
| EA20 | 126 - 45             |
| EA22 | 13 - 40              |
| EA23 | 16 - 35              |
| EA24 | 22 - 35              |

The inhibitory effect of antibody on complementation in this system appears to relate to the size of the deletion in the enzyme-acceptor. Enzyme-acceptor EA22 which deletes amino acids 13-40 and is the largest deletion tested in this experiment was inhibited least by antibody. Enzyme-acceptor, EA14 which deletes amino acids 30-37 is the smallest of the tested group and was inhibited the most by antibody. EA 20 which comprises amino acids 26-45 and is intermediate in size between EA22 and EA14 was relatively moderately inhibited. The native complementation efficiency of EA20 is, however, lower than that of either EA14 or EA22. The enzyme-acceptor must satisfy two criteria: (a) native complementation efficiency e.g., EA14 and EA22 are more efficient than other sequences based on equimolar concentrations; and (b) the ability of specific analyte-binding protein to inhibit complementation.

EXAMPLE: EFFECT OF A SECOND ANTIBODY ON THE DIGOXIN ENZYME IMMUNOASSAY

The results presented above suggest that coupling of anti-digoxin antibody to the enzyme-donor-digoxin conjugates of the present invention slows down the rate of complementation of enzyme-acceptor and enzyme-donor conjugate. Such coupling, however, does not completely prevent complementation. Thus, as stated in above the system has greatest sensitivity at approximately 15 minutes incubation of enzyme-acceptor and enzyme-donor conjugate.

The system reaches maximum absorbance differential at approximately 15 minutes. At that time the concentration of β-galactosidase in a system with anti-digoxin antibody present is the same as that in a system in which the antibody is absent, or in which digoxin antibody is neutralized (e.g., high digoxin levels). Since the β-galactosidase concentration is the same, the rate of substrate conversion is the same. No additional absorbance differential occurs. This phenomenon, which limits the effectiveness of the antibody on complementation produces a narrow absorbance range for a dose-response curve, flat slope characteristics and inadequate sensitivity of the assay for some diagnostic applications.

27

The following example demonstrates that attachment of a secondary antibody, specific for the anti-digoxin conjugate antibody, enhances the inhibition of complementation.

Attachment of Whole Secondary Antibody

In one series of experiments, 50 μl of rabbit anti-digoxin antibody (diluted 1:1000) was combined in a set of microtiter wells with 50 μl of digoxin-H6 (diluted 1:50 in PM2 buffer), and 50 μl of digoxin, in concentration of 0, 1, 2.5, 5, 7.5, 10, 100 ng/ml. A 50μl aliquot of a secondary antibody preparation (Bethyl Lab, Montgomery, TX, goat anti-rabbit serum 1:50-1:800) was added to each well. Results are tabulated in Tables VI and VII.

EP 0 419 081 A2

TABLE VI

EFFECT OF SECONDARY ANTIBODY ON RATE OF SUBSTRATE CONVERSION

| Dilution Secondary Antibody[b] | | 0-16 Minutes | | 16-30 Minutes | | 35-40 Minutes | | 45-60 Minutes | |
|---|---|---|---|---|---|---|---|---|---|
| | | OD/Time | % Prim. | OD/Time | % Prim. | OD/Time | % Prim. | OD/Time | % Prim. |
| 1:50 | .002 | 67 | .004 | 57 | .005 | 39 | .006 | 40 | |
| 1:75 | .002 | 67 | .004 | 57 | .007 | 54 | .009 | 60 | |
| 1:100 | .002 | 67 | .006 | 86 | .009 | 69 | .011 | 73 | |
| 1:200 | .003 | 100 | .007 | 100 | .012 | 92 | .013 | 87 | |
| 1:300 | .003 | 100 | .007 | 100 | .012 | 92 | .014 | 93 | |
| 1:400 | .003 | 100 | .007 | 100 | .013 | 100 | .014 | 93 | |
| 1:800 | .003 | 100 | .007 | 100 | .013 | 100 | .014 | 93 | |

RATE OF SUBSTRATE CONVERSION ($OD_{414}$/TIME) [a]

(a) In all cases the rate of substrate conversion by the assay system using primary antibody without the secondary antibody was desginated 100%. The measured rate for this preparation ($OD$/$_{414}$/Time) was: .003, .007, .013, .015 at 0-16; 16-30; 30-45; and 45-60 minutes, respectively.

(b) Secondary antibody used was goat anti-rabbit antibody (Bethyl Labs, Montgomery TX). All dilutions were prepared using PM2 Buffer. The primary antibody was rabbit anti-digoxin antibody diluted in all cases 1:1000.

As demonstrated in Table VI, the inhibitory effect on complementation achieved by attaching a secondary antibody to the antibody-digoxin H6 conjugate is optimal at a 1:50 dilution or less of the secondary antibody. At a dilution of 1:200 to 1:300 of the secondary antibody, all synergistic inhibition is lost. Thus, inhibition of complementation with or without secondary antibody is the same at this dilution or greater.

TABLE VII

| EFFECT OF SECONDARY ANTIBODY ON SUBSTRATE CONVERSION | | | | | | | | | |
|---|---|---|---|---|---|---|---|---|---|
| | | RATE OF SUBSTRATE CONVERSION | | | | | | | |
| | | 0-16 Minutes | | 16-30 Minutes | | 35-40 Minutes | | 45-60 Minutes | |
| Primary Antibody[a] | Dilution Secondary Antibody[b] | OD/Time | % Max. | OD/Time | % Max. | OD/Time | % Max. | OD/Time | % Max. |
| Rα Dg | --- | .002 | 50 | .007 | 70 | .010 | 71 | .010 | 77 |
| NRS[b] | --- | .004 | 100 | .010 | 100 | .014 | 100 | .013 | 100 |
| Rα Dg* | 1:5 | .004 | 100 | .007 | 54 | .003 | 23 | .003 | 23 |
| NRS | 1:5 | .004 | 100 | .013 | 100 | .013 | 100 | .013 | 100 |
| Rα Dg* | 1:10 | .004 | 100 | .005 | 39 | .003 | 21 | .003 | 21 |
| NRS | 1:10 | .004 | 100 | .013 | 100 | .014 | 100 | .014 | 100 |
| Rα Dg* | 1:20 | .003 | 60 | .003 | 25 | .003 | 21 | .003 | 21 |
| NRS | 1:20 | .005 | 100 | .012 | 100 | .014 | 100 | .014 | 100 |
| Rα Dg* | 1:40 | .002 | 40 | .003 | 25 | .003 | 21 | .004 | 29 |
| NRS | 1:40 | .005 | 100 | .012 | 100 | .014 | 100 | .014 | 100 |
| Rα Dg* | 1:80 | .001 | 20 | .004 | 36 | .006 | 43 | .006 | 43 |
| NRS | 1:80 | .005 | 100 | .011 | 100 | .014 | 100 | .014 | 100 |
| Rα Dg* | 1:160 | .002 | 50 | .006 | 50 | .009 | 60 | .009 | 60 |
| NRS | 1:160 | .004 | 100 | .012 | 100 | .015 | 100 | .015 | 100 |

\* Precipitation noted in wells

[a] Primary antibody was either rabbit anti-digoxin (Rα Dg*) or normal rabbit serum (NRS) diluted 1:1000 with PM2 Buffer.

[b] Secondary antibody designates a goat anti-rabbit antibody.

As demonstrated in Table VII, with no secondary antibody, the rate of substrate conversion (i.e., $\beta$-galactosidase concentration) reached 70% of maximum within 30 minutes. With secondary antibody at a 1:40 dilution, the rate of substrate conversion was approximately 25% of maximum. At greater than 1:40 dilutions of secondary antibody, the inhibitory effect on complementation diminished as evidenced by increasing rates of substrate conversion over time.

At dilutions equal to or greater than 1:40, the effect is an increase in the rate of substrate conversion.

At all concentrations of secondary antibody tested, the rate of substrate conversion (i.e., $\beta$-galactosidase concentration) became linear (i.e., no new $\beta$-galactosidase produced) at levels below the maximum concentration of $\beta$-galactosidase the system would permit (e.g., NRS replaces secondary antibody). This indicates that binding of secondary antibody enhances steric interference of the primary antibody and may completely prevent complementation by that enzyme-donor population which is bound.

Attachment of Fragment of Secondary Antibody

In order to determine the enhanced inhibition, observed when a secondary antibody was coupled to the primary antibody-enzyme-donor conjugate could be attributed to steric hinderance or entrapment of enzyme-donor conjugate in a precipitin complex, monovalent Fab fragments (antigen binding fragments about 50,000 daltons MW) of goat anti-rabbit immunoglobulin were used as the secondary antibody.

Because Fab fragments cannot cross link antigen they are not capable of inducing a precipitin or an agglutination reaction. Any inhibition of complementation observed in this preparation is due to enhanced steric effects on complementation and not to enhanced entrapment of conjugate.

In a microtiter plate format, five equal additions of 50 $\mu l$ each sequentially of digoxin (0, 1, 4, 10, 1000 ng/ml); digoxin-H6 conjugate; rabbit anti-digoxin primary antibody (1:4000) and secondary goat anti-rabbit antibody (Bethyl Labs, Montgomery, TX) at 1:80 dilution. All dilutions were in PM2 Buffer. The secondary antibody was replaced by normal rabbit serum (1:80) and the Fab fragment of goat anti-rabbit serum (Cappel Laboratories, West Chester, PA) at dilutions of 1:10, 1:20, 1:40, 1:80, 1:160, 1:320. After 10 minutes at room temperature 50 $\mu l$ of $1X10^{-5}M$ EA14 and 5 mg/ml of the substrate ONPG were added and incubations continued 30 minutes at 37°C. $OD_{414}$ was measured and Bound/Maximum Boun (B/Bma) determined.

The results are demonstrated in Table VIII.

TABLE VIII

| EFFECT OF FAB FRAGMENTS | | | | | |
|---|---|---|---|---|---|
| | Rate of Substrate Conversion (B/B MAX) Concentration Digoxin (ng/ml) | | | | |
| Dilution of Secondary Antibody Preparation[a] | 0 | 1 | 4 | 10 | 1000 |
| Goat anti-rabbit IgG[b] | 56.7 | 66.3 | 81.9 | 94.0 | 100 |
| None[c] | 95.4 | 91.3 | 88.7 | 97.1 | 100 |
| Fab 1:10 Goat anti-rabbit IgG[d] | 62.8 | 68.1 | 75.2 | 89.4 | 100 |
| Fab 1:20 Goat anti-rabbit IgG[d] | 57.6 | 67.6 | 75.5 | 87.1 | 100 |
| Fab 1:40 Goat anti-rabbit IgG[d] | 66.2 | 73.8 | 80.0 | 90.3 | 100 |
| Fab 1:80 Goat anti-rabbit IgG[d] | 69.7 | 75.6 | 82.3 | 91.5 | 100 |
| Fab 1:160 Goat anti-rabbit IgG[d] | 77.4 | 78.5 | 83.1 | 92.11 | 100 |
| Fab 1:320 Goat anti-rabbit IgG[d] | 81.6 | 83.5 | 85.1 | 94.8 | 100 |

[a] All secondary antibody preparations were tested using primary antibody at 1:4000 dilution.

[b] Goat anti-rabbit immunoglobulin antiserum (Bethyl Labs, Montgomery, TX).

[c] None designates that no secondary antibody was used. A 1:80 dilution of normal rabbit serum replaced the secondary antibody in these samples.

[d] Fab Goat anti-rabbit IgG designates the Fab fragment obtained from H and L Sp. (Cappel 0412-0081 Lot #23167)(Cappel Laboratories, West Chester, PA).

As demonstrated in Table VIII, decreased complementation is evident when the goat anti-rabbit immunoglobulin (Fab fragment) is coupled to the primary antibody enzyme-donor conjugate. The inhibition of complementation induced by the Fab fragment is approximately equivalent to that inhibition observed when using whole antibody.

As shown in Table VIII, the secondary antibody had a greater inhibiting effect on complementation at low dose (i.e., greater antibody/enzyme-donor interaction be caused by excess free analyte).

Decreasing Fab concentration did produce a linear decline in complementation inhibition. In Table VI intact molecules demonstrated a decrease in secondary antibody effectiveness with dilution greater than 1:40. Likewise, the same phenomenon is seen beginning with 1:40 dilution of the Fab preparation.

Inhibition of Complementation by Analyte-Specific Antibodies: A Comparison of ED-Digoxin Conjugates

Comparison of various ED-digoxin conjugates for specific inhibition of complementation activity by specific analyte antibodies was performed. In the experiment shown below, the complementation activity of the enzyme-donor coupled with EA22 was normalized. Digoxin-conjugates of the various ED's were prepared as previously described except for ED4 (2-digoxin) where the pH of coupling was raised to pH 9.5 to couple the digoxin-maleimide to both the $\alpha$-amino group and the cysteine distal to the $\alpha$-region. Anti-digoxin antibody and goat anti-rabbit antibody concentrations were both normalized. The results are shown in Table IX.

TABLE IX

| INHIBITION OF COMPLEMENTATION BY ANALYTE-SPECIFIC ANTIBODIES | |
| --- | --- |
| Enzyme-Donor | % Inhibition of Complementation |
| ED5 | 66 |
| ED4 | 68 |
| ED4 | 51 |
| H6 | 37 |

Improved Thyroxine and Digoxin Assays Utilizing Secondary Antibody

Thyroxine and digoxin enzyme complementation immunoassays were performed with secondary antibody.

The thyroxine (T4) assay was further refined with EA22 and ED4 on a centrifugal analyzer, the ENCORE® from Baker Instruments (Allentown, PA). The assay system consisted of 10 $\mu$l of patient sample, 100 $\mu$l of enzyme-acceptor reagent which also contained anti-T4 antibody and salicylate, and 290 $\mu$l of enzyme-donor reagent which also contained secondary goat anti-rabbit antibody and the substrate o-nitro-phenyl-$\beta$-D-galactopyranoside (ONPG). The final system concentrations were as follows:

| enzyme-acceptor (EA22) | $0.625 \times 10^{-7}$ M |
| --- | --- |
| 1° T4 antibody | 1/1200 |
| salicylate | 10 nM |
| enzyme-donor (ED4-T4) | 1/276 |
| 2° goat anti-rabbit antibody | 1/200 |
| ONPG | 0.51 mg/ml |

Readings were taken at 900 seconds. When various patient T4 samples are used, changes in OD/minute must be plotted due to ± 50 MOD input at $OD_{405}$ from individual patient samples. A T4 assay with calibrators prepared in whole human serum was performed. Changes in absorbance between calibrators at 900 seconds are plotted versus serum T4 concentrations.

The digoxin assay was refined using ED5 and EA22 and the Baker ENCORE® centrifugal analyzer. Digoxin standards were prepared in human serum. The assay consisted of 30 $\mu$l of sera, 200 $\mu$l of EDS-digoxin reagent and 100 $\mu$l of the EA22 reagent. The ED5-digoxin reagent also contained substrate o-nitrophenyl-$\beta$-D-galactopyranoside and goat anti-rabbit antibody. The EA22 reagent contained rabbit anti-digoxin antibody. The final system concentrations were as follows:

| serum | 9.1% |
|---|---|
| EA22 | $2 \times 10^{-7}$ |
| ED5-digoxin | 1:1500 |
| 1° digoxin antibody | 1:59,400 |
| 2° goat anti-rabbit antibody | 1:200 |
| ONPG | 0.5 mg/ml |

A standard curve for this assay was obtained

Comparison of Performance of Genetically Engineered and Chemically Synthesized Enzyme-Donors in Digoxin Immunoassay

To compare enzyme immunoassays performed with chemically synthesized versus genetically engineered components, two analogous enzyme-donors were prepared, one by recombinant DNA techniques and the other by chemical peptide synthesis. The amino acid sequences of ED3 created by genetic engineering and ED3A created by polypeptide synthesis are as follows:

ED3

```
Asp Pro Ser Gly Asp Pro Arg Ala Cys Ser Asn Ser Leu Ala
Val Val Leu Gln Arg Arg Asp Trp Glu Asn Pro Gly Val Thr
Glu Leu Asn Arg Leu Ala Ala His Pro Pro Phe Ala Ser Trp
Arg Asn Ser Glu Glu Ala Arg Thr Asp Arg Pro Ser Gln Gln
Leu Arg Ser Leu Asn Gly Leu Glu Ser Arg Ser Ala Gly Met
Pro Leu Glu
```

ED3A

```
Cys Ile Thr Asp Ser Leu Ala Val Val Leu Gln Arg Arg Asp
Trp Glu Asn Pro Gly Val Thr Gln Leu Asn Arg Leu Ala Ala
His Pro Pro Phe Ala Ser Trp Arg Asn Ser Glu Glu Ala Arg
Thr
```

The salient features of these two peptides are the analogous cysteine residue (Cys), marked with an asterisk used for chemical coupling to a analyte and the analogous a-donor domain, which in ED3 is located between amino acids number 12 and 50, inclusive, and in ED3A is located between amino acids number 5 and 43 inclusive, and correspond to amino acids 6 through 44 of wild-type-$\beta$-galactosidase.

Conjugation of digoxin to ED3 and ED3A was performed with 3-O-[maleimidophenylcarbamyl]-digoxigenin as described. Preparations of ED3, ED3A, digoxin-ED3 and digoxin-ED3A were subjected to high performance liquid chromatography (HPLC) on a preparative HPLC phenyl column (Waters $\mu$Bondapak, Waters Assoc., Milford, MA) using a gradient of 0.80% acetonitrile in water containing 0.1% TFA as eluent. Column fractions of each enzyme-donor were assayed for complementation as described above using M15 as enzyme-acceptor. The relative complementation efficiency of ED3-digoxin was four times greater than ED3A-digoxin.

Column fractions corresponding to digoxin-ED3 and digoxin-ED3A were pooled separately and compared in a competitive enzyme immunoassay for digoxin.

A 96-well microtiter plate was used for the assay. The assay comprised 25 $\mu$l of human serum standards 0, 0.5, 1, 2, 4, 10, 100 and 1000 ng/ml digoxin, 100 $\mu$l of reagent I which contains $4 \times 10^{-7}$M

M15 enzyme-acceptor and digoxin antibody, and 130 μl of reagent II. Reagent II contained various dilutions of digoxin-ED3 or digoxin-ED3A, secondary goat anti-rabbit antibody and 1.1 mg/ml of o-nitrophenyl-β-D-galatopyranoside. The results following a 30 minute incubation at 37° C and reading at 405 nm in a Titertek microtiter plate reader are shown in Table X, competitive immunoassays were created with both digoxin-ED3 and digoxin-ED3A. The digoxin immunoassay with digoxin-ED3 gave better signal discrimination at the low doses of 0.5 and 1 ng/ml than digoxin-ED3A. This discrepancy may be due to the presence of impurities in the ED3A preparation which were detected during HPLC analysis.

These experiments demonstrate the applicability of synthesized polypeptides, as well as genetically engineered polypeptides, in the control of the complementation of β-galactosidase polypeptides by antigen-antibody reaction. Hence, chemical polypeptide synthesis can be used to create enzyme-donors for the purpose of detecting high molecular weight proteins. Gene fusions that encode immunologically reactive polypeptide epitopes fused to the α-donor domain can also be synthesized. The limits on this approach include not only the state-of-the-art capability to synthesize ever larger polypeptides but also knowledge of the sequence of both the required α-donor domain and the immunologically reactive protein domain.

TABLE X

| DIGOXIN ASSAY WITH ED3 AND ED3A | | | | | | | | |
|---|---|---|---|---|---|---|---|---|
| Conjujugate Dilution | Digoxin Dose ng/ml | | | | | | | |
| | 0 | 0.5 | 1 | 2 | 4 | 10 | 100 | 1K |
| ED3 (Absolute OD) | | | | | | | | |
| 1/100 | .810 | .821 | .855 | .916 | .980 | 1.088 | 1.159 | 1.218 |
| 1/200 | .350 | .368 | .386 | .420 | .444 | .508 | .566 | .586 |
| 1/400 | .154 | .149 | .163 | .178 | .189 | .230 | .249 | .264 |
| 1/800 | .080 | .078 | .084 | .095 | .090 | .114 | .133 | .121 |
| ED3A (Absolute OD) | | | | | | | | |
| 1/100 | .668 | .656 | .660 | .660 | .668 | .719 | .757 | .777 |
| 1/200 | .310 | .306 | .309 | .322 | .329 | .352 | .372 | .375 |
| 1/400 | .146 | .146 | .153 | /152 | .151 | .192 | .180 | .180 |
| 1/800 | .043 | .039 | .047 | .043 | .048 | .060 | .064 | .052 |
| ED3 (ΔOD) | | | | | | | | |
| 1/100 | 0 | .011 | .045 | .106 | .170 | .278 | .349 | .408 |
| 1/200 | 0 | .018 | .036 | .070 | .094 | .158 | .216 | .236 |
| 1/400 | 0 | .005 | .009 | .024 | .035 | .076 | .095 | .110 |
| 1/800 | 0 | .002 | .004 | .015 | .010 | .034 | .053 | .041 |
| ED3A (ΔOD) | | | | | | | | |
| 1/100 | | -.012 | -.008 | -.008 | 0 | .051 | .089 | .104 |
| 1/200 | | -.004 | -.001 | -.012 | .019 | .042 | .062 | .065 |
| 1/400 | | 0 | .007 | .006 | .005 | .046 | .034 | .034 |

Deposit of Microorganisms

The following E . coli strains carrying the listed plasmids have been deposited with In Vitro International, Inc. (IVI) (Ann Arbor, MI) and have been assigned the following accession numbers:

34

| E. coli | Plasmid | Accession Numbers |
|---------|---------|-------------------|
| E9001 | p122 | IVI 10034 |
| E9001 | p125 | IVI 10035 |
| E9001 | pF29 | IVI 10038 |
| JM83 | p150 | IVI 10036 |
| JM83 | p157 | IVI 10037 |
| AMA 1004 | pMG14 | IVI 10050 |
| AMA 1004 | pMG22 | IVI 10051 |
| E9001 | p169 | IVI 10052 |
| E9001 | p183 | IVI 10053 |
| E9001 | p185 | IVI 10054 |

E . coli strain E9001, IVI 10034 and strain JM83, IVI 10037 contain plasmids pl22 and pl57, respectively, carrying genes coding for fusion proteins of part of the hepatitis B virus surface antigen and an α-donor, E . coli strain E9001, IVI 10035 contains plasmid p125 carrying a gene coding for an enzyme-donor. E . coli strain E9001, IVI 10038 and strain JM83, IVI 10036 contain plasmids pF29 and pl50, respectively, p150 carries a gene which codes for an enzyme-acceptor. E . coli strain AMA 1004, IVI 10050 contains a plasmid, pMG14, which carries a gene for a $\beta$-galactosidase protein (enzyme-acceptor) with amino acids 30-37 deleted. E . coli strain AMA 1004, IVI 10051 contains a plasmid, pMG22, which carries a gene for a $\beta$-galactosidase protein (enzyme-acceptor) with amino acids 13-40 deleted. E . coli strain E9001, IVI 10052 contains pl69, a plasmid which carries a gene coding for a fragment (ED H6) of $\beta$-galactosidase which has a cysteine residue at amino acid 62 and a lysine residue at amino acid 64. E . coli strain E9001, IVI 10053 contains pl83, a plasmid which carries a gene coding for a fragment (ED3) of 5-galactosidase which has cysteine residue at amino acid 3. E . coli strain E9001, IVI 10054 contains pl85, a plasmid which carries a gene coding for a fragment (ED5) of $\beta$-galactosidase which has a cysteine residue at amino acid 39.

A number of additional enzyme donor sequences were prepared, where the basic sequences as follows and the following table indicates the substitutions and deletions for the enzyme donor sequences.

```
    1           5           10          15          20          25
    M  D  P  S  G  N  P  Y  G  I  D  P  T  Q  S  S  P  G  N  I  D  P  R  A  S  S  N  S


    30          35          40          45          50          55
    L  A  V  V  L  G  R  R  D  W  E  N  P  G  V  T  Q  L  N  R  L  A  A  H  P  P  F  A


    60          65          70          75          80
    S  W  R  N  S  E  E  A  R  T  D  R  P  S  Q  Q  L  R  S  L  N  G  L  E  S  R  S  A


    85
    G  M  P  L  E
```

TABLE XI

| Site of Amino Acid Substitution with | | | |
|---|---|---|---|
| ED No. | cys | lys | Deletion or Substitution * |
| 3 | 3 | | D |
| 4 | 46 | | S |
| 5 | 39 | | D |
| 6 | 3 | | |
| 7 | 3, 46 | | D, S |
| 8 | 20 | | D |
| 9 | 46 | | D |
| 10 | 26 | | |
| 11 | 39 | | |
| 12 | 23 | | |
| 13 | | 3 | D |
| 14 | | 46 | |
| 16 | | 3 | |
| 18 | | 20 | D |
| 19 | 39 | | |
| 24 | 46 | | S |
| 28 | | 1, 46 | S |
| 50 | | 20 | |
| 57 | 46 | 17 | |
| 73 | 46 | 3 | |
| 74 | | 20 | |

*D = deletion of amino acids 6-20; S = A-Q-P-E-W in place of R-S-L-N

Various enzyme donors were conjugated to thyroxine and/or digoxin. Following the procedures described previously, a number of conjugates were prepared, with the following table indicating the conjugates.

TABLE XII

| Thyroxine Conjugates ED No. | Digoxin Conjugates ED No. |
|---|---|
| 3 | 3 |
| 4 | 4 |
| 5 | 6 |
| 6 | 8 |
| 7 | 11 |
| 8 | 13 |
| 9 | 14 |
| 11 | 16 |
| 12 | 50 |
| 28 | |
| 50 | |
| 57 | |

The enzyme donor conjugates were characterized by determining the immunopurity of the conjugate;

the kinetic specific activity; the percent inhibition at half saturation; the maximum amount of complementation inhibition and the affinity constant for antibody to the ligand. The assay conditions, except where other wise indicated are as follows:

The reagents employed were sample buffer: 100 mM sodium phosphate, 20 mM sodium azide and 0.2% BSA; assay buffer pH 7.0: 10 mM ethylene glycol, tetraacetic acid, 2 mM Mg acetate, 20 mM sodium azide, 150 mM sodium phosphate, 100 mM potassium phosphate, 0.05% Tween 20, 0.05 mM dithiothreitol.

Immunopurity was determined by precipitation of the enzyme donor conjugate with anti-T4 antibody TgG Sorbheads. The ED is incubated with primary antibody at room temperature for 15 min on a rocker shaker. Secondary antibody is added and incubation continued for 15 min. The suspension is centrifuged and an aliquot of the supernatant is saved for the assay. The above cycle is repeated on the remaining supernatant for a total of five cycles. A control sample of the ED has no primary antibody. The ED concentration during incubation is 20 nM. Assay concentrations are EA 500 nM; ED, 4 nM; and ONPG, 1mg/ml. Immunopurity is expressed as percent of ED activity precipitable by primary antibody.

The kinetic specific activity is determined using a COBAS BIO assay with pre-incubation of the ED with excess EA for 10 min. System concentrations are: ED 0.1-0.5 nM, EA 22, 500 nM and CPRG, 1.11 mM. Six ED concentrations in the above range were assayed in duplicate at 37°C. Data of units per assay versus ng protein/assay are plotted and a specific activity (u/mg) is calculated by least squares fitting. One unit is defined as 1 $\mu$mole CPRG/min.

The immunochemical testing was performed on the Encore. A two reagent format was employed, the first reagent contained the assay buffer, EA22 and ONPG, while the second reagent contained the ED, anti-T4 or anti-digoxin antibody and the concentrations were EA, 560 mM; ED, 2.4 nM; ONPG, 0.58 ng/ml. The ligand concentrations employed are indicated in the legend to the table. The ED and antibody were preincubated on the rotor for 15 min at 37°C. All antibody concentrations were assayed in duplicate. No correction was made for variations of total protein in the assays.

The Ka was determined as follows: To determine the affinity constant of an antibody for an experimental ED conjugate, an ED titration against a fixed antibody is carried out. Antibody binding to the conjugated ED is monitored by inhibition of formation of active enzyme as indicated by a decrease in chromogenic substrate turnover. The resulting data is plotted in a Scatchard format (Bound/Free vs. [Bound]). The slope of the line generated is equal to ~$K_A$ in L/mole.

The following table indicates the results.

## TABLE XIII

### CHARACTERIZATION OF ED CONJUGATES

| ED Conjugate | 1 Cys Pos. | 2 Amino Acid Length | 3 Immuno-Purity | 4 Specific Activity (u/mg) (% ED4-T4) Kinetic | Equilibrium | 5 %I 1/2Sat | 6 Immunochemical Testing (0.125 nM Sys [ED]) | 7 Ka (L/mole) x10-9 |
|---|---|---|---|---|---|---|---|---|
| ED3T4 | 3 | 74 | 100% | 99% | 90% | 28% | 41% | 1.12 |
| ED6T4 | 3 | 89 | 100% | 81% | 112% | 17% | 27% | 0.99 |
| ED57T4 | 17 | 89 | 75% | 15% | 36% | 0 | --- | ---- |
| ED8T4 | 20 | 74 | 90% | 93% | 95% | 30% | 42% | 0.93 |
| ED50T4 | 20 | 89 | 92% | 108% | 96% | 27% | 39% | 1.09 |
| ED12T4 | 23 | 89 | 94% | 43% | 61% | 39% | --- | 1.40 |
| ED5T4 | 39 | 74 | 93% | 53% | 138% | 0 | 0 | ---- |
| ED11T4 | 39 | 89 | 100% | 25% | 95% | 0 | 0 | ---- |
| ED9T4 | 46 | 74 | 100% | 78% | 67% | 22% | 30% | 1.89 |
| ED4T4 | 46 | 90 | 96% | 100% | 100% | 34% | 47% | 1.97 |
| ED24T4 | 46 | 89 | 97% | 100% | 100% | 29% | 35% | 5.09 |
| ED7T4 | 3 & 46 | 75 | 67% | 2% | 28% | 0% | 0% | ---- |
| ED3-Dig | 3 | 74 | 97% | 48% | 67% | 49% | | 2.15 |
| ED6-Dig | 3 | 89 | 98% | 42% | 80% | 34% | | 1.20 |
| ED8-Dig | 20 | 74 | 95% | 40% | 49% | 39% | | 2.70 |
| ED50-Dig | 20 | 89 | 97% | 27% | 72% | 31% | | 1.20 |

TABLE XIII Continued

| ED Conjugate | 1 Cys Pos. | 2 Amino Acid Length | 3 Immuno- Purity | 4 Specific Activity (u/mg) (% ED4-T4) | | 5 6 Immunochemical Testing %I (0.125 nM 1/2Sat Sys [ED]} | 7 Ka (L/mole) x10-9 |
|---|---|---|---|---|---|---|---|
| | | | | Kinetic | Equilibrium | | |
| ED5-Dig | 39 | 74 | 93% | 22% | 115% | 50% | 3.50 |
| ED11-Dig | 39 | 89 | 93% | 53% | 72% | 54% | 1.50 |
| ED4-Dig | 46 | 90 | 95% | 100% | 100% | 52% | 3.60 |
| ED24-Dig | 46 | 89 | 95% | 51% | 68% | 36% | 2.60 |

1) Cys position refers to the location of the unique Cys residue in the ED sequence. The numbering system is taken from the sequence of native b-galactosidase. 2) There are two lengths of ED molecules. The "short" version (74-75 amino acids) and the "long" version (89-90 amino acids), which is identical to the former except for the insertion of 15 amino acids in the amino terminal portion of the sequence between amino acids 5 and 6 of the short version. The inserted sequence is N-P-Y-G-I-D-P-T-Q-S-S-P-G-N-I. 3) Immunopurity is the % of ED conjugate activity which can be removed by analyte specific antiserum. 4) Kinetic specific activity is a measure of an EDs ability to form active enzyme by combining with EA in a 15 minute time frame relative to ED4. Equilibrium specific activity provides a measure of the amount of active enzyme formed after ED and EA have been incubated together for 18 hours. 5) %I at a half-saturation is the amount of complementation inhibition achieved when a standard Ab (T4-pool 393A, Digoxin-Cambridge 3429F) is held at a fixed dilution (T4-1:12,000; Dig 1:10,000 final) and ED is titrated until one-half of the Ab binding sites are saturated. 6) The maximum amount of complementation inhibition is approached when the ED system concentration is lowered to 0.125 nM, while holding Ab concentration constant. 7) Affinity constant derived form a Scatchard plot of an ED titration against a fixed Ab concentration. All measurements were made in the absence of secondary antibody.

Immunoassay For Folate

Preparation of ED14-Folate Conjuate

Folic acid (5.0 mg) dicyclohexylcarbodiimide (2.0 mg) and N-hydroxysuccinimide (1.0 mg) were dissolved in DMF (0.5 ml) with warming and stirred for 3 h. All of the folic acid did not dissolve. The solution was microfuged and the supernatant (200 $\mu$l) was added to ED14 (202 $\mu$g) in 0.2 M borate pH 8.5 (750 $\mu$l) and dimethylformamide (250 $\mu$l). The mixture was gently stirred for one hour, then microfuged in an Eppendorf tube. The supernatant was loaded on a G-25 column (Pharmacia) and eluted with PMJ-1. (One liter of PMJ-1 was prepared as follows: $KH_2PO_4$ (2.5 g), $K_2HPO_4$ (23.0 g), $NaH_2PO_4$, (12.5 g), ethylene-diaminetetraacetic acid, disodium salt (0.67 g), magnesium acetate-tetrahydrate (1.29 g), sodium azide (1.3 g) and ethylene glycol (24.5 ml) were dissolved in enough water to bring the total volume to 1.0 L.) The complementing fractions were combined and injected onto an HPLC (RP analytical phenyl $\mu$ Bondapak column, linear 1% gradient, Buffer A: 0.1% TFA in $H_2O$; Buffer B: 0.1% TFA, 80% $CH_3CN$ in $H_2O$). The conjugate eluted at 34.1 minutes followed by unreacted ED14 at 34.5 minutes. The first fraction was collected, combined and stored at 4°C.

Procedure for Folic Acid Assay

The following buffer systems and reagents were used and will be abbreviated as stated below:
1. Sample buffer; 100 mM sodium phosphate, 20 mM sodium azide and 0.2% BSA.
2. Assay buffer pH 7.0; 10 mM ethylene glycol tetraacetic acid, 2 mM magnesium acetate, 20 mM sodium azide, 150 mM sodium phosphate, 100 mM potassium phosphate, 0.05% Tween 20, 0.05 mM dithiothreitol, adjusted to pH 7.0.
3. EA; enzyme acceptor
4. ED; enzyme donor
5. FBP Scripps; folate binding protein from Scripps 2.25 mg/ml stock
6. CPRG; chlorophenol red $\beta$-D galactopyranoside
7. ONPG: 2-nitrophenol $\beta$-D galactopyranoside

The ED14-folate conjugates were evaluated for complementation with EA to active enzyme. Two ED14-folate fractions, #34 and *35 were titrated with varying EA concentrations. The following reagents were employed; EA aG concentrations of $6.0 \times 10^{-6}$M, $1.2 \times 10^{-5}$M, $2.0 \times 10^{-5}$M, $3.0 \times 10^{-5}$M, in Assay buffer pH 7.0, ED14-folate (fractions #34 and #35) at concentrations of $7.5 \times 10^{-9}$M in assay/CPRG buffer pH 7.0, and sample buffer. The following protocol was used on the Encore analyzer (Baker Instruments); 41.6 $\mu$l of EA reagent was combined with 41.6 $\mu$l of sample buffer in the upper well of the Encore rotor, while 166.7 $\mu$l of the ED14-folate was loaded into the lower well. Rates were calculated by subtraction at 2 min, from the $OD_{580}$ at 3 min. The results are shown in Table XIII. As the EA concentration increases, the enzyme activity of the system also increases.

TABLE IV

| | EA Reagent | EA Final | mAu*/Min Rate 2'-3' | mAu*/Min S.D. | EA/ED |
|---|---|---|---|---|---|
| ED14-FOL #34 | $6 \times 10^{-6}$ | $1 \times 10^{-6}$ | 490.8 | 18.4 | 200 |
| Reagent $7.5 \times 10^{-9}$ M | $1.2 \times 10^{-5}$ | $2 \times 10^{-6}$ | 782.2 | 10.5 | 400 |
| Final $5 \times 10^{-9}$ M | $2.0 \times 10^{-5}$ | $3.3 \times 10^{-6}$ | 967.2 | 22.4 | 667 |
| | $3.0 \times 10^{-5}$ | $5.0 \times 10^{-6}$ | 1045 | 23.8 | 1000 |
| ED14-FOL #35 | $6 \times 10^{-6}$ | $1 \times 10^{-6}$ | 579 | 19.3 | 200 |
| Reagent $7.5 \times 10^{-9}$ M | $1.2 \times 10^{-5}$ | $2 \times 10^{-6}$ | 920 | 29.9 | 400 |
| Final $5 \times 10^{-9}$ M | $2.0 \times 10^{-5}$ | $3.3 \times 10^{-6}$ | 1086 | 17.0 | 667 |
| | $3.0 \times 10^{-5}$ | $5.0 \times 10^{-6}$ | 1186 | 2.3 | 1000 |

*mAu = milli(absorption units)

The ability of FBP to inhibit complementation of ED14-folate to EA was evaluated. Fraction #34 of ED14-folate was titrated against varying concentrations of FBP. The following reagents were used; EA at a concentration of $1.5 \times 10^{-6}$ in Assay buffer at pH 7.0, ED14-folate at a concentration of $7.7 \times 10^{-9}$ M in Assay/CPRG buffer pH 7.0, FBP at dilutions of 1:10, 1:20, 1:40, 1:60, 1:80, 1:120, 1:160, 1:200, 1:240, 1:300, 1:400, 1:600, 1:800 in sample buffer. The following protocol was used on the Baker Instruments Encore analyzer; 41.6 $\mu$l of EA reagent was combined with 41.6 $\mu$l of sample buffer in the upper well of the Encore rotor, while 162 $\mu$l of ED14-folate was mixed with 5 $\mu$l of diluted FBP and incubated for 25 min at room temperature and then loaded into the lower well of the Encore rotor. Rates were calculated by subtracting $OD_{580}$ at 9 min from $OD_{580}$ at 11 min. The results are shown in Table XIV.

A folate dose response was elicited as follows. The following reagents were prepared; EA at a concentration of 3750 nM in Assay buffer p8 7.0, ED14-folate (fraction #34) at a concentration of 26.66 nM in Assay/ONPG buffer pH 7.0, FBP at a dilution of 1:1000 in sample buffer and folic acid standards at 0, 2.5, 5.0, 10.0 and 20.0 ng/ml in sample buffer. The following protocol was used on the Encore analyzer (Baker Instruments); 25 $\mu$l of folic acid standard and 25$\mu$l of FBP were combined and incubated for 30 min at room temperature, the sample-FBP mixture was then added to 150 $\mu$l of 26.66 nM ED14-folate and incubated for 30 min at room temperature with 200 $\mu$l being loaded into the lower well of the Encore rotor, 50 $\mu$l of the 3750 nM EA solution was loaded into the upper well of the Encore rotor. Rates were calculated by subtracting the $OD_{420}$ at 5 min from the $OD_{420}$ at 9 min.

| ng/ml | 5'- 9' | Net | % Mod |
|---|---|---|---|
| 0 | 336.5 | -- | -- |
| 2.5 | 362 | 25.5 | 7.0 |
| 5.0 | 388.5 | 52 | 13.4 |
| 10.0 | 443.5 | 107 | 24.1 |
| 20.0 | 562 | 2255 | 40.0 |
| The result is a straight line with r = 0.999, m = 11.32, and y = 333.6. | | | |

TABLE XV

| FBP Dil Reagent | FBP Dil Final | mAu*/Min Rate 9 -11 | % Inhibition |
|---|---|---|---|
| 1:10 | 1:500 | 274 | 67.5 |
| 1:20 | 1:1000 | 278 | 67.0 |
| 1:40 | 1:2K | 295 | 64.7 |
| 1:60 | 1:3K | 298 | 64.7 |
| 1:80 | 1:4K | 306.5 | 63.7 |
| 1:120 | 1:6K | 330.5 | 60.8 |
| 1:160 | 1:8K | 358 | 57.6 |
| 1:200 | 1:10K | 362.5 | 57.0 |
| 1:240 | 1:12K | 403 | 52.2 |
| 1:300 | 1:15K | 429 | 49.1 |
| 1:400 | 1:20K | 499.5 | 41.8 |
| 1:600 | 1:30K | 595.5 | 29.4 |
| 1:800 | 1:40K | 637.5 | 24.4 |

*mAu = milli(absorption units)

It is evident from the above results that the subject method provides for a sensitive and accurate assay, which may be employed in a variety of protocols. In addition, storage stability is greatly enhanced, since the fragments are quite stable and are readily activated upon combination in an aqueous medium. The individual fragments are easily conjugated to a wide variety of ligands of interest, to provide for active conjugates which result in a broad dynamic range for the varicus ligands.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. An enzyme assay method for determining the amount of a suspected analyte in a sample, where said analyte is a member of a specific binding pair consisting of ligand and receptor, which method comprises the steps of:

(a) forming a reaction mixture by combining in a medium (1) sample; (2) an enzyme donor polypeptide comprising the mutated N-proximal fragment of β-galactosidase, wherein from 2 to 3 amino acids are substituted with cysteine or lysine to provide a site for conjugation with a ligand immunologically cross-reactive with said analyte or complementary to receptor analyte (3) an analyte binding protein, when said-analyte is other than a receptor; and (4) an enzyme acceptor polypeptide consisting essentially of a C-proximal fragment of β-galactosidase, and said enzyme donor and said enzyme acceptor form an active enzyme complex having β-galactosidase activity, which activity is distinguishable when a receptor is bound to said conjugate;

(b) measuring the rate of conversion of substrate in the reaction mixture; and

(c) determining the amount of analyte in the sample by comparing the rate of conversion of substrate to a rate of conversion of substrate obtained using a known amount of analyte.

2. A method according to Claim 1, wherein said enzyme donor is mutated to cysteine for conjugation to a ligand.

3. A method according to Claim 1, wherein said enzyme donor is mutated to lysine for conjugation to a ligand.

4. A method according to Claim 1, wherein one of said mutations is at amino acid 46.

5. A method according to Claim 4, wherein one of said mutations is in the region of amino acids 1 to 5.

42

6. A method according to Claim 1, wherein said mutations are at other then amino acids 48 to 61.

7. A method according to Claim 1, wherein said analyte is a hapten.

8. A method according to Claim 1, wherein said analyte is an antigen.

9. A method according to Claim 1, wherein said reaction mixture further comprises (5) an antibody that binds said receptor.

10. A protein composition having at least 40 amino acids of the following sequence:

```
1*  *  * 5         10          15
M D P S G N P Y G I D P T Q S

   .      20      *  25*      30
S P G N I D P R A S S N S L A

        *35        *40** * * *45*
V V L Q R R D W E N P G V T Q
                        20

*    *  50   *    55*      60
L N R L A A H P P F A S W R N

*      65   *  70        75
S E E A R T·D R P S Q Q L R S
40                      50

        80        85 *  89
L N G L E S R S A G M P L G
   56
```

wherein the numbers underneath letters indicate the wild-type β-galactosidase numbering and an asterisk indicates a site wherein from 2 to 3 of the amino acids indicated with asterisks are substituted with cysteine or lysine.

11. A protein composition according to Claim 10, wherein said substitution is at positions 1 to 5 or 46.

12. A protein composition according to Claim 10, having a deletion of at least one amino acid in the region amino acids 5 to 20.

13. A protein composition according to Claim 30, wherein the sequence R-S-L-N is substituted with the sequence A-E-P-E-W.

14. A protein composition according to Claim 10, wherein said protein composition is covalently bonded to a ligand.

15. A protein composition according to Claim 14, wherein said ligand is digoxin, thyroxine, a drug of abuse, a therapeutic drug, a steroid, or an immunologically cross-reactive compound thereof.

16. A recombinant DNA vector comprising a replication system functional in a microorganism host and an expression cassette comprising transcriptional and translational initiation and termination regulatory regions functional in said host, and a open reading frame encoding a protein composition according to Claim 10.

17. A bacterium comprising a vector according to Claim 16.

18. A bacterium according to Claim 17, wherein said bacterium is E . coli .

19. A kit for use in an assay according to Claim 1, comprising enzyme donor, enzyme acceptor, wherein said enzyme donor and enzyme acceptor complex to form an active β-galactosidase, receptor for said ligand when said analyte is ligand, and optionally β-galactosidase substrate.